# EUROPEAN PATENT APPLICATION

(11) **EP 2 447 277 A1**
(43) Date of publication of application: **02.05.2012**
(21) Application number: 10382284.7
(22) Date of filing: 28.10.2010
(51) Int. Cl.: C07K 14/16, A61K 39/21

(54) **Vaccine compositions based on modified gp41 immunogens**

(71) Applicant: Laboratorios del. Dr. Esteve, S.A., 08041 Barcelona (ES); Fundació Privada Institut de Recerca de la SIDA-Caixa, 08916 Badalona (ES)
(72) Inventor: Blanco Arbúes, Julián Miguel, E-08500 Vic (ES); Carrillo Molina, Jorge, E-08915 Badalona (ES); Curriu Martí, Marta, E-43004 Tarragona (ES)
(74) Representative: ABG Patentes, S.L.

(57) **Abstract**

The invention relates to relates to human immunodeficiency virus (HIV) immunogens based on gp41 epitopes located in the membrane proximal extracellular region (MPER). The immunogens of the present invention are capable of inducing the expression of neutralizing antibodies. Various forms of these isolated immunogens are disclosed and exemplified. Isolated nucleic acid molecules expressing relevant portions of these immunogens, as well as the vectors and host cells utilized for their production, are also within the scope of the present invention.

SEQUENCE LISTING

<110>  Laboratorios del Dr. Esteve S.A.
       Fundació Privada Institut de Recerca de la SIDA-Caixa

<120>  VACCINE COMPOSITIONS BASED ON MODIFIED gp41 IMMUNOGENS

<130>  P6150EP00

<160>  37

<170>  PatentIn version 3.5

<210>  1
<211>  30
<212>  PRT
<213>  Human immunodeficiency virus

<400>  1

Ala Val Gly Ile Gly Ala Leu Phe Leu Gly Phe Leu Gly Ala Ala Gly
1               5                   10                  15

Ser Thr Met Gly Ala Ala Ser Met Thr Leu Thr Val Gln Ala
            20                  25                  30

<210>  2
<211>  52
<212>  PRT
<213>  Human immunodeficiency virus

<400>  2

Arg Gln Leu Leu Ser Gly Ile Val Gln Gln Gln Asn Asn Leu Leu Arg
1               5                   10                  15

Ala Ile Glu Ala Gln Gln His Leu Leu Gln Leu Thr Val Trp Gly Ile
            20                  25                  30

Lys Gln Leu Gln Ala Arg Ile Leu Ala Val Glu Arg Tyr Leu Lys Asp
        35                  40                  45

Gln Gln Leu Leu
    50

<210>  3
<211>  28
<212>  PRT
<213>  Human immunodeficiency virus

<400>  3

Gly Ile Trp Gly Cys Ser Gly Lys Leu Ile Cys Thr Thr Ala Val Pro
1               5                   10                  15

Trp Asn Ala Ser Trp Ser Asn Lys Ser Leu Glu Gln
            20                  25

<210>  4
<211>  29
<212>  PRT
<213>  Human immunodeficiency virus

<400>  4

Gly Ile Trp Gly Cys Ser Gly Lys Leu Ile Cys Thr Thr Ala Val Pro
1               5                   10                  15

Trp Asn Ala Ser Trp Ser Asn Lys Ser Leu Glu Gln Ile
            20                  25

<210>  5
<211>  29
<212>  PRT
<213>  Human immunodeficiency virus

<400>  5

Gly Ile Trp Gly Cys Ser Gly Lys Leu Ile Cys Thr Thr Ala Val Pro
1               5                   10                  15

Trp Asn Ala Ser Trp Ser Asn Lys Ser Leu Glu Gln Ile
            20                  25

<210>  6
<211>  861
<212>  PRT
<213>  Human immunodeficiency virus

<400>  6

Met Arg Val Lys Glu Lys Tyr Gln His Leu Trp Arg Trp Gly Trp Lys
1               5                   10                  15

Trp Gly Thr Met Leu Leu Gly Ile Leu Met Ile Cys Ser Ala Thr Glu
            20                  25                  30

Lys Leu Trp Val Thr Val Tyr Tyr Gly Val Pro Val Trp Lys Glu Ala
        35                  40                  45

Thr Thr Thr Leu Phe Cys Ala Ser Asp Ala Lys Ala Tyr Asp Thr Glu
    50                  55                  60

Val His Asn Val Trp Ala Thr His Ala Cys Val Pro Thr Asp Pro Asn
65                  70                  75                  80

Pro Gln Glu Val Val Leu Val Asn Val Thr Glu Asn Phe Asn Met Trp
                85                  90                  95

Lys Asn Asp Met Val Glu Gln Met His Glu Asp Ile Ile Ser Leu Trp
            100                 105                 110

Asp Gln Ser Leu Lys Pro Cys Val Lys Leu Thr Pro Leu Cys Val Ser
        115                 120                 125

Leu Lys Cys Thr Asp Leu Gly Asn Ala Thr Asn Thr Asn Ser Ser Asn
    130                 135                 140

Thr Asn Ser Ser Ser Gly Glu Met Met Met Glu Lys Gly Glu Ile Lys
145                 150                 155                 160

Asn Cys Ser Phe Asn Ile Ser Thr Ser Ile Arg Gly Lys Val Gln Lys
                165                 170                 175

Glu Tyr Ala Phe Phe Tyr Lys Leu Asp Ile Ile Pro Ile Asp Asn Asp
            180                 185                 190

Thr Thr Ser Tyr Thr Leu Thr Ser Cys Asn Thr Ser Val Ile Thr Gln
        195                 200                 205

Ala Cys Pro Lys Val Ser Phe Glu Pro Ile Pro Ile His Tyr Cys Ala
    210                 215                 220

Pro Ala Gly Phe Ala Ile Leu Lys Cys Asn Asn Lys Thr Phe Asn Gly
225                 230                 235                 240

Thr Gly Pro Cys Thr Asn Val Ser Thr Val Gln Cys Thr His Gly Ile
                245                 250                 255

Arg Pro Val Val Ser Thr Gln Leu Leu Leu Asn Gly Ser Leu Ala Glu
            260                 265                 270

Glu Glu Val Val Ile Arg Ser Ala Asn Phe Thr Asp Asn Ala Lys Thr
        275                 280                 285

Ile Ile Val Gln Leu Asn Gln Ser Val Glu Ile Asn Cys Thr Arg Pro
    290                 295                 300

Asn Asn Asn Thr Arg Lys Ser Ile Arg Ile Gln Arg Gly Pro Gly Arg
305                 310                 315                 320

Ala Phe Val Thr Ile Gly Lys Ile Gly Asn Met Arg Gln Ala His Cys
                325                 330                 335

Asn Ile Ser Arg Ala Lys Trp Asn Ala Thr Leu Lys Gln Ile Ala Ser
            340                 345                 350

Lys Leu Arg Glu Gln Phe Gly Asn Asn Lys Thr Ile Ile Phe Lys Gln
        355                 360                 365

Ser Ser Gly Gly Asp Pro Glu Ile Val Thr His Ser Phe Asn Cys Gly
    370                 375                 380

Gly Glu Phe Phe Tyr Cys Asn Ser Thr Gln Leu Phe Asn Ser Thr Trp
385                 390                 395                 400

Phe Asn Ser Thr Trp Ser Thr Glu Gly Ser Asn Asn Thr Glu Gly Ser
                405                 410                 415

Asp Thr Ile Thr Leu Pro Cys Arg Ile Lys Gln Phe Ile Asn Met Trp
            420                 425                 430

Gln Glu Val Gly Lys Ala Met Tyr Ala Pro Pro Ile Ser Gly Gln Ile
        435                 440                 445

Arg Cys Ser Ser Asn Ile Thr Gly Leu Leu Leu Thr Arg Asp Gly Gly
    450                 455                 460

Asn Asn Asn Asn Gly Ser Glu Ile Phe Arg Pro Gly Gly Gly Asp Met
465                 470                 475                 480

Arg Asp Asn Trp Arg Ser Glu Leu Tyr Lys Tyr Lys Val Val Lys Ile
                485                 490                 495

Glu Pro Leu Gly Val Ala Pro Thr Lys Ala Lys Arg Arg Val Val Gln
            500                 505                 510

Arg Glu Lys Arg Ala Val Gly Ile Gly Ala Leu Phe Leu Gly Phe Leu
        515                 520                 525

Gly Ala Ala Gly Ser Thr Met Gly Ala Arg Ser Met Thr Leu Thr Val
    530                 535                 540

Gln Ala Arg Gln Leu Leu Ser Gly Ile Val Gln Gln Gln Asn Asn Leu
545                 550                 555                 560

Leu Arg Ala Ile Glu Ala Gln Gln His Leu Leu Gln Leu Thr Val Trp
                565                 570                 575

Gly Ile Lys Gln Leu Gln Ala Arg Ile Leu Ala Val Glu Arg Tyr Leu
            580                 585                 590

Lys Asp Gln Gln Leu Leu Gly Ile Trp Gly Cys Ser Gly Lys Leu Ile
        595                 600                 605

Cys Thr Thr Ala Val Pro Trp Asn Ala Ser Trp Ser Asn Lys Ser Leu
    610                 615                 620

Glu Gln Ile Trp Asn Asn Met Thr Trp Met Glu Trp Asp Arg Glu Ile
625                 630                 635                 640

Asn Asn Tyr Thr Ser Leu Ile His Ser Leu Ile Glu Glu Ser Gln Asn
                645                 650                 655

Gln Gln Glu Lys Asn Glu Gln Glu Leu Leu Glu Leu Asp Lys Trp Ala
            660                 665                 670

Ser Leu Trp Asn Trp Phe Asn Ile Thr Asn Trp Leu Trp Tyr Ile Lys
        675                 680                 685

Ile Phe Ile Met Ile Val Gly Gly Leu Val Gly Leu Arg Ile Val Phe
    690                 695                 700

Ala Val Leu Ser Ile Val Asn Arg Val Arg Gln Gly Tyr Ser Pro Leu
705                 710                 715                 720

Ser Phe Gln Thr His Leu Pro Thr Pro Arg Gly Pro Asp Arg Pro Glu
                725                 730                 735

Gly Ile Glu Glu Glu Gly Gly Glu Arg Asp Arg Asp Arg Ser Ile Arg
            740                 745                 750

Leu Val Asn Gly Ser Leu Ala Leu Ile Trp Asp Asp Leu Arg Ser Leu
        755                 760                 765

Cys Leu Phe Ser Tyr His Arg Leu Arg Asp Leu Leu Leu Ile Val Thr
    770                 775                 780

Arg Ile Val Glu Leu Leu Gly Arg Arg Gly Trp Glu Ala Leu Lys Tyr
785                 790                 795                 800

Trp Trp Asn Leu Leu Gln Tyr Trp Ser Gln Glu Leu Lys Asn Ser Ala
                805                 810                 815

Val Ser Leu Leu Asn Ala Thr Ala Ile Ala Val Ala Glu Gly Thr Asp
            820                 825                 830

Arg Val Ile Glu Val Val Gln Gly Ala Cys Arg Ala Ile Arg His Ile
        835                 840                 845

Pro Arg Arg Ile Arg Gln Gly Leu Glu Arg Ile Leu Leu
    850                 855                 860

<210>  7
<211>  80
<212>  PRT
<213>  ARTIFICIAL SEQUENCE

<220>
<223>  GP41-MIN

<400>  7

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu Glu Leu Asp Lys Trp Ala Ser
        35                  40                  45

Leu Trp Asn Trp Phe Asn Ile Thr Asn Trp Leu Trp Tyr Ile Lys Leu
    50                  55                  60

Phe Ile Met Ile Val Gly Gly Leu Val Gly Leu Arg Ile Val Phe Ala
65                  70                  75                  80

<210>  8
<211>  133
<212>  PRT
<213>  ARTIFICIAL SEQUENCE

<220>
<223>  GP41-MIN with a tetrad glycine linker and a V5 vector epitope tag
       at the C-terminus

<400>  8

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu Glu Leu Asp Lys Trp Ala Ser
        35                  40                  45

Leu Trp Asn Trp Phe Asn Ile Thr Asn Trp Leu Trp Tyr Ile Lys Leu
    50                  55                  60

Phe Ile Met Ile Val Gly Gly Leu Val Gly Leu Arg Ile Val Phe Ala
65                  70                  75                  80

Val Leu Ser Ile Val Asn Arg Ala Gly Gly Gly Gly Lys Gly Gln Asp
                85                  90                  95

Asn Ser Ala Asp Ile Gln His Ser Gly Gly Arg Ser Ser Leu Glu Gly
            100                 105                 110

Pro Arg Phe Glu Gly Lys Pro Ile Pro Asn Pro Leu Leu Gly Leu Asp
        115                 120                 125

Ser Thr Arg Thr Gly
    130

<210>  9
<211>  139
<212>  PRT
<213>  ARTIFICIAL SEQUENCE

<220>
<223>  GP41-MIN with a hexahistidine tag at the C-terminus

<400>  9

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu Glu Leu Asp Lys Trp Ala Ser
        35                  40                  45

Leu Trp Asn Trp Phe Asn Ile Thr Asn Trp Leu Trp Tyr Ile Lys Leu
    50                  55                  60

Phe Ile Met Ile Val Gly Gly Leu Val Gly Leu Arg Ile Val Phe Ala
65                  70                  75                  80

Val Leu Ser Ile Val Asn Arg Ala Gly Gly Gly Gly Lys Gly Gln Asp
                85                  90                  95

Asn Ser Ala Asp Ile Gln His Ser Gly Gly Arg Ser Ser Leu Glu Gly
            100                 105                 110

Pro Arg Phe Glu Gly Lys Pro Ile Pro Asn Pro Leu Leu Gly Leu Asp
        115                 120                 125

Ser Thr Arg Thr Gly His His His His His His
    130                 135

<210>  10
<211>  236
<212>  PRT
<213>  ARTIFICIAL SEQUENCE

<220>
<223>  GP41-MINFULL

<400>  10

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu Glu Leu Asp Lys Trp Ala Ser
        35                  40                  45

Leu Trp Asn Trp Phe Asn Ile Thr Asn Trp Leu Trp Tyr Ile Lys Leu
    50                  55                  60

Phe Ile Met Ile Val Gly Gly Leu Val Gly Leu Arg Ile Val Phe Ala
65                  70                  75                  80

Val Leu Ser Ile Val Asn Arg Val Arg Gln Gly Tyr Ser Pro Leu Ser
                85                  90                  95

Phe Gln Thr His Leu Pro Thr Pro Arg Gly Pro Asp Arg Pro Glu Gly
            100                 105                 110

Ile Glu Glu Glu Gly Gly Glu Arg Asp Arg Asp Arg Ser Ile Arg Leu
        115                 120                 125

Val Asn Gly Ser Leu Ala Leu Ile Trp Asp Asp Leu Arg Ser Leu Cys
    130                 135                 140

Leu Phe Ser Tyr His Arg Leu Arg Asp Leu Leu Leu Ile Val Thr Arg
145                 150                 155                 160

Ile Val Glu Leu Leu Gly Arg Arg Gly Trp Glu Ala Leu Lys Tyr Trp
                165                 170                 175

Trp Asn Leu Leu Gln Tyr Trp Ser Gln Glu Leu Lys Asn Ser Ala Val
            180                 185                 190

Ser Leu Leu Asn Ala Thr Ala Ile Ala Val Ala Glu Gly Thr Asp Arg
        195                 200                 205

Val Ile Glu Val Val Gln Gly Ala Cys Arg Ala Ile Arg His Ile Pro
    210                 215                 220

Arg Arg Ile Arg Gln Gly Leu Glu Arg Ile Leu Leu
225                 230                 235

<210>  11
<211>  281
<212>  PRT
<213>  ARTIFICIAL SEQUENCE

<220>
<223>  GP41-MINFULL with a tetrad glycine linker and a V5 vector epitope
       tag at the C-terminus

<400>  11

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu Glu Leu Asp Lys Trp Ala Ser
        35                  40                  45

Leu Trp Asn Trp Phe Asn Ile Thr Asn Trp Leu Trp Tyr Ile Lys Leu
    50                  55                  60

Phe Ile Met Ile Val Gly Gly Leu Val Gly Leu Arg Ile Val Phe Ala
65                  70                  75                  80

Val Leu Ser Ile Val Asn Arg Val Arg Gln Gly Tyr Ser Pro Leu Ser
                85                  90                  95

Phe Gln Thr His Leu Pro Thr Pro Arg Gly Pro Asp Arg Pro Glu Gly
            100                 105                 110

Ile Glu Glu Glu Gly Gly Glu Arg Asp Arg Asp Arg Ser Ile Arg Leu
        115                 120                 125

Val Asn Gly Ser Leu Ala Leu Ile Trp Asp Asp Leu Arg Ser Leu Cys
    130                 135                 140

Leu Phe Ser Tyr His Arg Leu Arg Asp Leu Leu Leu Ile Val Thr Arg
145                 150                 155                 160

Ile Val Glu Leu Leu Gly Arg Arg Gly Trp Glu Ala Leu Lys Tyr Trp
                165                 170                 175

Trp Asn Leu Leu Gln Tyr Trp Ser Gln Glu Leu Lys Asn Ser Ala Val
            180                 185                 190

Ser Leu Leu Asn Ala Thr Ala Ile Ala Val Ala Glu Gly Thr Asp Arg
        195                 200                 205

Val Ile Glu Val Val Gln Gly Ala Cys Arg Ala Ile Arg His Ile Pro
    210                 215                 220

Arg Arg Ile Arg Gln Gly Leu Glu Arg Ile Leu Leu Gly Gly Gly Gly
225                 230                 235                 240

Lys Gly Gln Asp Asn Ser Ala Asp Ile Gln His Ser Gly Gly Arg Ser
                245                 250                 255

Ser Leu Glu Gly Pro Arg Phe Glu Gly Lys Pro Ile Pro Asn Pro Leu
            260                 265                 270

Leu Gly Leu Asp Ser Thr Arg Thr Gly
        275                 280

<210>  12
<211>  287
<212>  PRT
<213>  ARTIFICIAL SEQUENCE

<220>
<223>  GP41-MINFULL with a hexahistidine tag at the C-terminus

<400>  12

Met Ile Trp Asn Asn Met Thr Trp Met Glu Trp Asp Arg Glu Ile Asn
1               5                   10                  15

Asn Tyr Thr Ser Leu Ile His Ser Leu Ile Glu Glu Ser Gln Asn Gln
            20                  25                  30

Gln Glu Lys Asn Glu Gln Glu Leu Leu Glu Leu Asp Lys Trp Ala Ser
        35                  40                  45

Leu Trp Asn Trp Phe Asn Ile Thr Asn Trp Leu Trp Tyr Ile Lys Leu
    50                  55                  60

Phe Ile Met Ile Val Gly Gly Leu Val Gly Leu Arg Ile Val Phe Ala
65                  70                  75                  80

Val Leu Ser Ile Val Asn Arg Val Arg Gln Gly Tyr Ser Pro Leu Ser
                85                  90                  95

Phe Gln Thr His Leu Pro Thr Pro Arg Gly Pro Asp Arg Pro Glu Gly
            100                 105                 110

Ile Glu Glu Glu Gly Gly Glu Arg Asp Arg Asp Arg Ser Ile Arg Leu
        115                 120                 125

Val Asn Gly Ser Leu Ala Leu Ile Trp Asp Asp Leu Arg Ser Leu Cys
    130                 135                 140

Leu Phe Ser Tyr His Arg Leu Arg Asp Leu Leu Leu Ile Val Thr Arg
145                 150                 155                 160

Ile Val Glu Leu Leu Gly Arg Arg Gly Trp Glu Ala Leu Lys Tyr Trp
                165                 170                 175

Trp Asn Leu Leu Gln Tyr Trp Ser Gln Glu Leu Lys Asn Ser Ala Val
            180                 185                 190

Ser Leu Leu Asn Ala Thr Ala Ile Ala Val Ala Glu Gly Thr Asp Arg
        195                 200                 205

Val Ile Glu Val Val Gln Gly Ala Cys Arg Ala Ile Arg His Ile Pro
    210                 215                 220

Arg Arg Ile Arg Gln Gly Leu Glu Arg Ile Leu Leu Gly Gly Gly Gly
225                 230                 235                 240

Lys Gly Gln Asp Asn Ser Ala Asp Ile Gln His Ser Gly Gly Arg Ser
                245                 250                 255

Ser Leu Glu Gly Pro Arg Phe Glu Gly Lys Pro Ile Pro Asn Pro Leu
            260                 265                 270

Leu Gly Leu Asp Ser Thr Arg Thr Gly His His His His His His
        275                 280                 285

<210>  13
<211>  116
<212>  PRT
<213>  ARTIFICIAL SEQUENCE

<220>
<223>  GP41-STAPLE

<400>  13

Met Ala Val Gly Ile Gly Ala Met Phe Leu Gly Phe Leu Gly Ala Ala
1               5                   10                  15

Gly Ser Thr Met Gly Ala Ala Ser Met Thr Leu Thr Val Ile Trp Asn
            20                  25                  30

His Thr Thr Trp Met Glu Trp Asp Arg Glu Ile Asn Asn Tyr Thr Ser
        35                  40                  45

Leu Ile His Ser Leu Ile Glu Glu Ser Gln Asn Gln Gln Glu Lys Asn
    50                  55                  60

Glu Gln Glu Leu Leu Glu Leu Asp Lys Trp Ala Ser Leu Trp Asn Trp
65                  70                  75                  80

Phe Asn Ile Thr Asn Trp Leu Trp Tyr Ile Lys Leu Phe Ile Met Ile
                85                  90                  95

Val Gly Gly Leu Val Gly Leu Arg Ile Val Phe Ala Val Leu Ser Ile
            100                 105                 110

Val Asn Arg Ala
        115

<210>  14
<211>  161
<212>  PRT
<213>  ARTIFICIAL SEQUENCE

<220>
<223>  GP41-MIN with a tetrad glycine linker and a V5 vector epitope tag
       at the C-terminus

<400>  14

Met Ala Val Gly Ile Gly Ala Met Phe Leu Gly Phe Leu Gly Ala Ala
1               5                   10                  15

Gly Ser Thr Met Gly Ala Ala Ser Met Thr Leu Thr Val Ile Trp Asn
            20                  25                  30

His Thr Thr Trp Met Glu Trp Asp Arg Glu Ile Asn Asn Tyr Thr Ser
        35                  40                  45

Leu Ile His Ser Leu Ile Glu Glu Ser Gln Asn Gln Gln Glu Lys Asn
    50                  55                  60

Glu Gln Glu Leu Leu Glu Leu Asp Lys Trp Ala Ser Leu Trp Asn Trp
65                  70                  75                  80

Phe Asn Ile Thr Asn Trp Leu Trp Tyr Ile Lys Leu Phe Ile Met Ile
                85                  90                  95

Val Gly Gly Leu Val Gly Leu Arg Ile Val Phe Ala Val Leu Ser Ile
            100                 105                 110

Val Asn Arg Ala Gly Gly Gly Gly Lys Gly Gln Asp Asn Ser Ala Asp
        115                 120                 125

Ile Gln His Ser Gly Gly Arg Ser Ser Leu Glu Gly Pro Arg Phe Glu
    130                 135                 140

Gly Lys Pro Ile Pro Asn Pro Leu Leu Gly Leu Asp Ser Thr Arg Thr
145                 150                 155                 160

Gly

<210>  15
<211>  167
<212>  PRT
<213>  ARTIFICIAL SEQUENCE

<220>
<223>  GP41-STAPLE with an additional hexahistidine tag at the
       C-terminus

<400>  15

Met Ala Val Gly Ile Gly Ala Met Phe Leu Gly Phe Leu Gly Ala Ala
1               5                   10                  15

Gly Ser Thr Met Gly Ala Ala Ser Met Thr Leu Thr Val Ile Trp Asn
            20                  25                  30

His Thr Thr Trp Met Glu Trp Asp Arg Glu Ile Asn Asn Tyr Thr Ser
        35                  40                  45

Leu Ile His Ser Leu Ile Glu Glu Ser Gln Asn Gln Gln Glu Lys Asn
    50                  55                  60

Glu Gln Glu Leu Leu Glu Leu Asp Lys Trp Ala Ser Leu Trp Asn Trp
65                  70                  75                  80

Phe Asn Ile Thr Asn Trp Leu Trp Tyr Ile Lys Leu Phe Ile Met Ile
                85                  90                  95

Val Gly Gly Leu Val Gly Leu Arg Ile Val Phe Ala Val Leu Ser Ile
            100                 105                 110

Val Asn Arg Ala Gly Gly Gly Gly Lys Gly Gln Asp Asn Ser Ala Asp
        115                 120                 125

Ile Gln His Ser Gly Gly Arg Ser Ser Leu Glu Gly Pro Arg Phe Glu
    130                 135                 140

Gly Lys Pro Ile Pro Asn Pro Leu Leu Gly Leu Asp Ser Thr Arg Thr
145                 150                 155                 160

Gly His His His His His His
                165

<210>  16
<211>  264
<212>  PRT
<213>  ARTIFICIAL SEQUENCE

<220>
<223>  GP41-STAPLEFULL

<400>  16

Met Ala Val Gly Ile Gly Ala Met Phe Leu Gly Phe Leu Gly Ala Ala
1               5                   10                  15

Gly Ser Thr Met Gly Ala Ala Ser Met Thr Leu Thr Val Ile Trp Asn
            20                  25                  30

His Thr Thr Trp Met Glu Trp Asp Arg Glu Ile Asn Asn Tyr Thr Ser
        35                  40                  45

Leu Ile His Ser Leu Ile Glu Glu Ser Gln Asn Gln Gln Glu Lys Asn
    50                  55                  60

Glu Gln Glu Leu Leu Glu Leu Asp Lys Trp Ala Ser Leu Trp Asn Trp
65                  70                  75                  80

Phe Asn Ile Thr Asn Trp Leu Trp Tyr Ile Lys Leu Phe Ile Met Ile
                85                  90                  95

Val Gly Gly Leu Val Gly Leu Arg Ile Val Phe Ala Val Leu Ser Ile
            100                 105                 110

Val Asn Arg Val Arg Gln Gly Tyr Ser Pro Leu Ser Phe Gln Thr His
        115                 120                 125

Leu Pro Thr Pro Arg Gly Pro Asp Arg Pro Glu Gly Ile Glu Glu Glu
    130                 135                 140

Gly Gly Glu Arg Asp Arg Asp Arg Ser Ile Arg Leu Val Asn Gly Ser
145                 150                 155                 160

Leu Ala Leu Ile Trp Asp Asp Leu Arg Ser Leu Cys Leu Phe Ser Tyr
                165                 170                 175

His Arg Leu Arg Asp Leu Leu Leu Ile Val Thr Arg Ile Val Glu Leu
            180                 185                 190

Leu Gly Arg Arg Gly Trp Glu Ala Leu Lys Tyr Trp Trp Asn Leu Leu
        195                 200                 205

Gln Tyr Trp Ser Gln Glu Leu Lys Asn Ser Ala Val Ser Leu Leu Asn
    210                 215                 220

Ala Thr Ala Ile Ala Val Ala Glu Gly Thr Asp Arg Val Ile Glu Val
225                 230                 235                 240

Val Gln Gly Ala Cys Arg Ala Ile Arg His Ile Pro Arg Arg Ile Arg
                245                 250                 255

Gln Gly Leu Glu Arg Ile Leu Leu
            260

<210>  17
<211>  309
<212>  PRT
<213>  ARTIFICIAL SEQUENCE

<220>
<223>  GP41-STAPLEFULL with a tetrad glycine linker and a V5 vector
       epitope tag at the C-terminus

<400>  17

Met Ala Val Gly Ile Gly Ala Met Phe Leu Gly Phe Leu Gly Ala Ala
1               5                   10                  15

Gly Ser Thr Met Gly Ala Ala Ser Met Thr Leu Thr Val Ile Trp Asn
            20                  25                  30

His Thr Thr Trp Met Glu Trp Asp Arg Glu Ile Asn Asn Tyr Thr Ser
        35                  40                  45

Leu Ile His Ser Leu Ile Glu Glu Ser Gln Asn Gln Gln Glu Lys Asn
    50                  55                  60

Glu Gln Glu Leu Leu Glu Leu Asp Lys Trp Ala Ser Leu Trp Asn Trp
65                  70                  75                  80

Phe Asn Ile Thr Asn Trp Leu Trp Tyr Ile Lys Leu Phe Ile Met Ile
                85                  90                  95

Val Gly Gly Leu Val Gly Leu Arg Ile Val Phe Ala Val Leu Ser Ile
            100                 105                 110

Val Asn Arg Val Arg Gln Gly Tyr Ser Pro Leu Ser Phe Gln Thr His
        115                 120                 125

Leu Pro Thr Pro Arg Gly Pro Asp Arg Pro Glu Gly Ile Glu Glu Glu
    130                 135                 140

Gly Gly Glu Arg Asp Arg Asp Arg Ser Ile Arg Leu Val Asn Gly Ser
145                 150                 155                 160

Leu Ala Leu Ile Trp Asp Asp Leu Arg Ser Leu Cys Leu Phe Ser Tyr
                165                 170                 175

His Arg Leu Arg Asp Leu Leu Leu Ile Val Thr Arg Ile Val Glu Leu
            180                 185                 190

Leu Gly Arg Arg Gly Trp Glu Ala Leu Lys Tyr Trp Trp Asn Leu Leu
        195                 200                 205

Gln Tyr Trp Ser Gln Glu Leu Lys Asn Ser Ala Val Ser Leu Leu Asn
    210                 215                 220

Ala Thr Ala Ile Ala Val Ala Glu Gly Thr Asp Arg Val Ile Glu Val
225                 230                 235                 240

Val Gln Gly Ala Cys Arg Ala Ile Arg His Ile Pro Arg Arg Ile Arg
                245                 250                 255

Gln Gly Leu Glu Arg Ile Leu Leu Gly Gly Gly Gly Lys Gly Gln Asp
            260                 265                 270

Asn Ser Ala Asp Ile Gln His Ser Gly Gly Arg Ser Ser Leu Glu Gly
        275                 280                 285

Pro Arg Phe Glu Gly Lys Pro Ile Pro Asn Pro Leu Leu Gly Leu Asp
    290                 295                 300

Ser Thr Arg Thr Gly
305

<210>  18
<211>  315
<212>  PRT
<213>  ARTIFICIAL SEQUENCE

<220>
<223>  GP41-STAPLEFULL with a hexahistidine tag at the C-terminus

<400>  18

Met Ala Val Gly Ile Gly Ala Met Phe Leu Gly Phe Leu Gly Ala Ala
1               5                   10                  15

Gly Ser Thr Met Gly Ala Ala Ser Met Thr Leu Thr Val Ile Trp Asn
            20                  25                  30

His Thr Thr Trp Met Glu Trp Asp Arg Glu Ile Asn Asn Tyr Thr Ser
        35                  40                  45

Leu Ile His Ser Leu Ile Glu Glu Ser Gln Asn Gln Gln Glu Lys Asn
    50                  55                  60

Glu Gln Glu Leu Leu Glu Leu Asp Lys Trp Ala Ser Leu Trp Asn Trp
65                  70                  75                  80

Phe Asn Ile Thr Asn Trp Leu Trp Tyr Ile Lys Leu Phe Ile Met Ile
                85                  90                  95

Val Gly Gly Leu Val Gly Leu Arg Ile Val Phe Ala Val Leu Ser Ile
            100                 105                 110

Val Asn Arg Val Arg Gln Gly Tyr Ser Pro Leu Ser Phe Gln Thr His
        115                 120                 125

Leu Pro Thr Pro Arg Gly Pro Asp Arg Pro Glu Gly Ile Glu Glu Glu
    130                 135                 140

Gly Gly Glu Arg Asp Arg Asp Arg Ser Ile Arg Leu Val Asn Gly Ser
145                 150                 155                 160

Leu Ala Leu Ile Trp Asp Asp Leu Arg Ser Leu Cys Leu Phe Ser Tyr
                165                 170                 175

His Arg Leu Arg Asp Leu Leu Leu Ile Val Thr Arg Ile Val Glu Leu
            180                 185                 190

Leu Gly Arg Arg Gly Trp Glu Ala Leu Lys Tyr Trp Trp Asn Leu Leu
        195                 200                 205

Gln Tyr Trp Ser Gln Glu Leu Lys Asn Ser Ala Val Ser Leu Leu Asn
    210                 215                 220

Ala Thr Ala Ile Ala Val Ala Glu Gly Thr Asp Arg Val Ile Glu Val
225                 230                 235                 240

Val Gln Gly Ala Cys Arg Ala Ile Arg His Ile Pro Arg Arg Ile Arg
                245                 250                 255

Gln Gly Leu Glu Arg Ile Leu Leu Gly Gly Gly Gly Lys Gly Gln Asp
            260                 265                 270

Asn Ser Ala Asp Ile Gln His Ser Gly Gly Arg Ser Ser Leu Glu Gly
        275                 280                 285

Pro Arg Phe Glu Gly Lys Pro Ile Pro Asn Pro Leu Leu Gly Leu Asp
    290                 295                 300

Ser Thr Arg Thr Gly His His His His His His
305                 310                 315

<210>  19
<211>  276
<212>  DNA
<213>  ARTIFICIAL SEQUENCE

<220>
<223>  GP41-MIN

<400>  19
atgatttgga ataacatgac ctggatggag tgggacagag aaattaacaa ttacacaagc       60

ttaatacact ccttaattga agaatcgcaa aaccagcaag aaaagaatga acaagaatta      120

ttggaattag ataaatgggc aagtttgtgg aattggttta acataacaaa ttggctgtgg 180

tacataaaat tattcataat gatagtagga ggcttggtag gtttaagaat agtttttgct 240

gtactttcta tagtgaatag agctggtggc ggtggc 276

<210> 20
<211> 399
<212> DNA
<213> ARTIFICIAL SEQUENCE

<220>
<223> GP41-MIN with a tetrad glycine linker and a V5 vector epitope tag

<400> 20
atgatttgga ataacatgac ctggatggag tgggacagag aaattaacaa ttacacaagc 60

ttaatacact ccttaattga agaatcgcaa aaccagcaag aaaagaatga acaagaatta 120

ttggaattag ataaatgggc aagtttgtgg aattggttta acataacaaa ttggctgtgg 180

tacataaaat tattcataat gatagtagga ggcttggtag gtttaagaat agtttttgct 240

gtactttcta tagtgaatag agctggtggc ggtggcaagg gtcaagacaa ttctgcagat 300

atccagcaca gtggcggccg ctcgagtcta gagggcccgc ggttcgaagg taagcctatc 360

cctaaccctc tcctcggtct cgattctacg cgtaccggt 399

<210> 21
<211> 417
<212> DNA
<213> ARTIFICIAL SEQUENCE

<220>
<223> GP41-MIN with a hexahistidine tag

<400> 21
atgatttgga ataacatgac ctggatggag tgggacagag aaattaacaa ttacacaagc 60

ttaatacact ccttaattga agaatcgcaa aaccagcaag aaaagaatga acaagaatta 120

ttggaattag ataaatgggc aagtttgtgg aattggttta acataacaaa ttggctgtgg 180

tacataaaat tattcataat gatagtagga ggcttggtag gtttaagaat agtttttgct 240

gtactttcta tagtgaatag agctggtggc ggtggcaagg gtcaagacaa ttctgcagat 300

atccagcaca gtggcggccg ctcgagtcta gagggcccgc ggttcgaagg taagcctatc 360

cctaaccctc tcctcggtct cgattctacg cgtaccggtc atcatcacca tcaccat 417

<210> 22
<211> 708

<212>  DNA
<213>  ARTIFICIAL SEQUENCE

<220>
<223>  GP41-MINFULL

<400>  22
atgatttgga ataacatgac ctggatggag tgggacagag aaattaacaa ttacacaagc      60

ttaatacact ccttaattga agaatcgcaa aaccagcaag aaaagaatga acaagaatta     120

ttggaattag ataaatgggc aagtttgtgg aattggttta acataacaaa ttggctgtgg     180

tatataaaat tattcataat gatagtagga ggcttggtag gtttaagaat agtttttgct     240

gtactttcta tagtgaatag agttaggcag ggatattcac cattatcgtt tcagacccac     300

ctcccaaccc cgaggggacc cgacaggccc gaaggaatag aagaagaagg tggagagaga     360

gacagagaca gatccattcg attagtgaac ggatccttgg cacttatctg ggacgatctg     420

cggagcctgt gcctcttcag ctaccaccgc ttgagagact tactcttgat tgtaacgagg     480

attgtggaac ttctgggacg cagggggtgg gaagccctca aatattggtg gaatctccta     540

cagtattgga gtcaggaact aaagaatagt gctgttagct tgctcaatgc cacagccata     600

gcagtagctg aggggacaga tagggttata gaagtagtac aaggagcttg tagagctatt     660

cgccacatac ctagaagaat aagacagggc ttggaaagga ttttgcta                  708


<210>  23
<211>  843
<212>  DNA
<213>  ARTIFICIAL SEQUENCE

<220>
<223>  GP41-MINFULL with a tetrad glycine linker and a V5 vector epitope
       tag

<400>  23
atgatttgga ataacatgac ctggatggag tgggacagag aaattaacaa ttacacaagc      60

ttaatacact ccttaattga agaatcgcaa aaccagcaag aaaagaatga acaagaatta     120

ttggaattag ataaatgggc aagtttgtgg aattggttta acataacaaa ttggctgtgg     180

tatataaaat tattcataat gatagtagga ggcttggtag gtttaagaat agtttttgct     240

gtactttcta tagtgaatag agttaggcag ggatattcac cattatcgtt tcagacccac     300

ctcccaaccc cgaggggacc cgacaggccc gaaggaatag aagaagaagg tggagagaga     360

gacagagaca gatccattcg attagtgaac ggatccttgg cacttatctg ggacgatctg     420

cggagcctgt gcctcttcag ctaccaccgc ttgagagact tactcttgat tgtaacgagg     480

attgtggaac ttctgggacg cagggggtgg gaagccctca aatattggtg gaatctccta 540

cagtattgga gtcaggaact aaagaatagt gctgttagct tgctcaatgc cacagccata 600

gcagtagctg aggggacaga tagggttata gaagtagtac aaggagcttg tagagctatt 660

cgccacatac ctagaagaat aagacagggc ttggaaagga ttttgctagg tggcggtggc 720

aagggtcaag acaattctgc agatatccag cacagtggcg gccgctcgag tctagagggc 780

ccgcggttcg aaggtaagcc tatccctaac cctctcctcg gtctcgattc tacgcgtacc 840

ggt 843

<210> 24
<211> 861
<212> DNA
<213> ARTIFICIAL SEQUENCE

<220>
<223> GP41-MINFULL with a hexahistidine tag

<400> 24
atgatttgga ataacatgac ctggatggag tgggacagag aaattaacaa ttacacaagc 60

ttaatacact ccttaattga agaatcgcaa aaccagcaag aaaagaatga acaagaatta 120

ttggaattag ataaatgggc aagtttgtgg aattggttta acataacaaa ttggctgtgg 180

tatataaaat tattcataat gatagtagga ggcttggtag gtttaagaat agtttttgct 240

gtactttcta tagtgaatag agttaggcag ggatattcac cattatcgtt tcagacccac 300

ctcccaaccc cgaggggacc cgacaggccc gaaggaatag aagaagaagg tggagagaga 360

gacagagaca gatccattcg attagtgaac ggatccttgg cacttatctg ggacgatctg 420

cggagcctgt gcctcttcag ctaccaccgc ttgagagact tactcttgat tgtaacgagg 480

attgtggaac ttctgggacg cagggggtgg gaagccctca aatattggtg gaatctccta 540

cagtattgga gtcaggaact aaagaatagt gctgttagct tgctcaatgc cacagccata 600

gcagtagctg aggggacaga tagggttata gaagtagtac aaggagcttg tagagctatt 660

cgccacatac ctagaagaat aagacagggc ttggaaagga ttttgctagg tggcggtggc 720

aagggtcaag acaattctgc agatatccag cacagtggcg gccgctcgag tctagagggc 780

ccgcggttcg aaggtaagcc tatccctaac cctctcctcg gtctcgattc tacgcgtacc 840

ggtcatcatc accatcacca t 861

<210> 25
<211> 360
<212> DNA

<213>  ARTIFICIAL SEQUENCE

<220>
<223>  GP41-STAPLE

<400>  25
atggcagtgg gaataggagc tatgttcctt gggttcttgg gagcagcagg aagcactatg      60

ggcgcagcct caatgacgct gacggtaatt tggaatcaca cgacctggat ggagtgggac     120

agagaaatta acaattacac aagcttaata cactccttaa ttgaagaatc gcaaaaccag     180

caagaaaaga atgaacaaga attattggaa ttagataaat gggcaagttt gtggaattgg     240

tttaacataa caaattggct gtggtatata aaattattca taatgatagt aggaggcttg     300

gtaggtttaa gaatagtttt tgctgtactt tctatagtga atagagctgg tggcggtggc     360


<210>  26
<211>  483
<212>  DNA
<213>  ARTIFICIAL SEQUENCE

<220>
<223>  GP41-STAPLE with a tetrad glycine linker and a V5 vector epitope
       tag

<400>  26
atggcagtgg gaataggagc tatgttcctt gggttcttgg gagcagcagg aagcactatg      60

ggcgcagcct caatgacgct gacggtaatt tggaatcaca cgacctggat ggagtgggac     120

agagaaatta acaattacac aagcttaata cactccttaa ttgaagaatc gcaaaaccag     180

caagaaaaga atgaacaaga attattggaa ttagataaat gggcaagttt gtggaattgg     240

tttaacataa caaattggct gtggtatata aaattattca taatgatagt aggaggcttg     300

gtaggtttaa gaatagtttt tgctgtactt tctatagtga atagagctgg tggcggtggc     360

aagggtcaag acaattctgc agatatccag cacagtggcg gccgctcgag tctagagggc     420

ccgcggttcg aaggtaagcc tatccctaac cctctcctcg gtctcgattc tacgcgtacc     480

ggt                                                                   483


<210>  27
<211>  501
<212>  DNA
<213>  ARTIFICIAL SEQUENCE

<220>
<223>  GP41-STAPLE with a hexahistidine tag

<400>  27
atggcagtgg gaataggagc tatgttcctt gggttcttgg gagcagcagg aagcactatg      60

ggcgcagcct caatgacgct gacggtaatt tggaatcaca cgacctggat ggagtgggac 120

agagaaatta acaattacac aagcttaata cactccttaa ttgaagaatc gcaaaaccag 180

caagaaaaga atgaacaaga attattggaa ttagataaat gggcaagttt gtggaattgg 240

tttaacataa caaattggct gtggtatata aaattattca taatgatagt aggaggcttg 300

gtaggtttaa gaatagtttt tgctgtactt tctatagtga atagagctgg tggcggtggc 360

aagggtcaag acaattctgc agatatccag cacagtggcg gccgctcgag tctagagggc 420

ccgcggttcg aaggtaagcc tatccctaac cctctcctcg gtctcgattc tacgcgtacc 480

ggtcatcatc accatcacca t 501

<210> 28
<211> 792
<212> DNA
<213> ARTIFICIAL SEQUENCE

<220>
<223> GP41-STAPLEFULL

<400> 28
atggcagtgg gaataggagc tatgttcctt gggttcttgg gagcagcagg aagcactatg 60

ggcgcagcct caatgacgct gacggtaatt tggaatcaca cgacctggat ggagtgggac 120

agagaaatta acaattacac aagcttaata cactccttaa ttgaagaatc gcaaaaccag 180

caagaaaaga atgaacaaga attattggaa ttagataaat gggcaagttt gtggaattgg 240

tttaacataa caaattggct gtggtatata aaattattca taatgatagt aggaggcttg 300

gtaggtttaa gaatagtttt tgctgtactt tctatagtga atagagttag gcagggatat 360

tcaccattat cgtttcagac ccacctccca accccgaggg gacccgacag gcccgaagga 420

atagaagaag aaggtggaga gagagacaga gacagatcca ttcgattagt gaacggatcc 480

ttggcactta tctgggacga tctgcggagc ctgtgcctct tcagctacca ccgcttgaga 540

gacttactct tgattgtaac gaggattgtg gaacttctgg gacgcagggg gtgggaagcc 600

ctcaaatatt ggtggaatct cctacagtat tggagtcagg aactaaagaa tagtgctgtt 660

agcttgctca atgccacagc catagcagta gctgagggga cagatagggt tatagaagta 720

gtacaaggag cttgtagagc tattcgccac atacctagaa gaataagaca gggcttggaa 780

aggattttgc ta 792

<210> 29
<211> 927

<212>  DNA
<213>  ARTIFICIAL SEQUENCE

<220>
<223>  GP41-STAPLEFULL with a tetrad glycine linker and a V5 vector
       epitope tag

<400>  29
atggcagtgg gaataggagc tatgttcctt gggttcttgg gagcagcagg aagcactatg        60

ggcgcagcct caatgacgct gacggtaatt tggaatcaca cgacctggat ggagtgggac       120

agagaaatta acaattacac aagcttaata cactccttaa ttgaagaatc gcaaaaccag       180

caagaaaaga atgaacaaga attattggaa ttagataaat gggcaagttt gtggaattgg       240

tttaacataa caaattggct gtggtatata aaattattca taatgatagt aggaggcttg       300

gtaggtttaa gaatagtttt tgctgtactt tctatagtga atagagttag gcagggatat       360

tcaccattat cgtttcagac ccacctccca accccgaggg gacccgacag gcccgaagga       420

atagaagaag aaggtggaga gagagacaga gacagatcca ttcgattagt gaacggatcc       480

ttggcactta tctgggacga tctgcggagc ctgtgcctct tcagctacca ccgcttgaga       540

gacttactct tgattgtaac gaggattgtg gaacttctgg gacgcagggg gtgggaagcc       600

ctcaaatatt ggtggaatct cctacagtat tggagtcagg aactaaagaa tagtgctgtt       660

agcttgctca atgccacagc catagcagta gctgagggga cagatagggt tatagaagta       720

gtacaaggag cttgtagagc tattcgccac atacctagaa gaataagaca gggcttggaa       780

aggattttgc taggtggcgg tggcaagggt caagacaatt ctgcagatat ccagcacagt       840

ggcggccgct cgagtctaga gggcccgcgg ttcgaaggta agcctatccc taaccctctc       900

ctcggtctcg attctacgcg taccggt                                           927


<210>  30
<211>  945
<212>  DNA
<213>  ARTIFICIAL SEQUENCE

<220>
<223>  GP41-STAPLEFULL with a hexahistidine tag

<400>  30
atggcagtgg gaataggagc tatgttcctt gggttcttgg gagcagcagg aagcactatg        60

ggcgcagcct caatgacgct gacggtaatt tggaatcaca cgacctggat ggagtgggac       120

agagaaatta acaattacac aagcttaata cactccttaa ttgaagaatc gcaaaaccag       180

caagaaaaga atgaacaaga attattggaa ttagataaat gggcaagttt gtggaattgg       240

tttaacataa caaattggct gtggtatata aaattattca taatgatagt aggaggcttg 300

gtaggtttaa gaatagtttt tgctgtactt tctatagtga atagagttag gcagggatat 360

tcaccattat cgtttcagac ccacctccca accccgaggg gacccgacag gcccgaagga 420

atagaagaag aaggtggaga gagagacaga gacagatcca ttcgattagt gaacggatcc 480

ttggcactta tctgggacga tctgcggagc ctgtgcctct tcagctacca ccgcttgaga 540

gacttactct tgattgtaac gaggattgtg gaacttctgg gacgcagggg gtgggaagcc 600

ctcaaatatt ggtggaatct cctacagtat tggagtcagg aactaaagaa tagtgctgtt 660

agcttgctca atgccacagc catagcagta gctgagggga cagatagggt tatagaagta 720

gtacaaggag cttgtagagc tattcgccac atacctagaa gaataagaca gggcttggaa 780

aggattttgc taggtggcgg tggcaagggt caagacaatt ctgcagatat ccagcacagt 840

ggcggccgct cgagtctaga gggcccgcgg ttcgaaggta agcctatccc taaccctctc 900

ctcggtctcg attctacgcg taccggtcat catcaccatc accat 945

<210> 31
<211> 28
<212> DNA
<213> ARTIFICIAL SEQUENCE

<220>
<223> primer GP41-EC-f forward amplifying the fusion peptide

<400> 31
caccatggca gtgggaatag gagctatg 28

<210> 32
<211> 23
<212> DNA
<213> ARTIFICIAL SEQUENCE

<220>
<223> Primer FP reverse amplifying the fusion peptide

<400> 32
taccgtcagc gtcattgagg ctg 23

<210> 33
<211> 29
<212> DNA
<213> ARTIFICIAL SEQUENCE

<220>
<223> Primer GP41-2L forward amplifying the HR1 region

<400> 33

caccatgcag gccagacaat tattgtctg                                    29

<210>  34
<211>  28
<212>  DNA
<213>  ARTIFICIAL SEQUENCE

<220>
<223>  Primer GP41-MAX forward amplifying the HR2 region

<400>  34
caccatgatt tggaataaca tgacctgg                                     28

<210>  35
<211>  31
<212>  DNA
<213>  ARTIFICIAL SEQUENCE

<220>
<223>  Primer HR-2 f-2 forward amplifying the HR2 region

<400>  35
gctgacggta atttggaatc acacgacctg g                                 31

<210>  36
<211>  31
<212>  DNA
<213>  ARTIFICIAL SEQUENCE

<220>
<223>  Primer GP41 reverse amplifying the transmembrane region

<400>  36
gccaccgcca cctagctcta ttcactatag a                                 31

<210>  37
<211>  29
<212>  DNA
<213>  ARTIFICIAL SEQUENCE

<220>
<223>  Primer GP41-MAX reverse amplifying the Intracellular region

<400>  37
gccaccgcca cctagcaaaa tcctttcca                                    29

## Description

### FIELD OF THE INVENTION

The present invention relates to human immunodeficiency virus (HIV) immunogens based on gp41 epitopes located in the membrane proximal extracellular region (MPER). The immunogens of the present invention are capable of inducing the expression of neutralizing antibodies. Various forms of these isolated immunogens are disclosed and exemplified. Isolated nucleic acid molecules expressing relevant portions of these immunogens, as well as the vectors and host cells utilized for their production, are also within the scope of the present invention.

### BACKGROUND OF THE INVENTION

It is estimated that more than 60 million people worldwide have been infected by the human immunodeficiency virus since 1982. Nearly half of these infected individuals have died of the resultant Acquired Immunodeficiency Syndrome (AIDS) during the same time frame. Although the virus spread seems to have reached a plateau lately, 2.7 million HIV new infections were reported in 2007. HIV still is a major public health problem. *See* UNAIDS, 2008 Report on the Global AIDS Epidemic.

HIV acts by targeting and infecting CD4+ T cells. By these means HIV disables a major immune response mechanism against not only the HIV infection, but also other opportunistic diseases. *See* Dalgleish A, et al., Nature 1984; 312:767-768; Maddon F, et al., Cell 1986; 47:333-348; McDougall J, et al., Science 1986; 231:382-385.

The infection of CD4+ T cells by HIV is characterized by the fusion of the HIV viral membrane and the target cellular membrane. This process is catalyzed by the envelope glycoprotein (env) gp160. The mature gp160 is composed of two non-covalently associated subunits, the surface gp120 and the transmembrane gp41 proteins. *See* Wyatt R, Sodroski J, Science 1998; 280:1884-1888.

The extracellular moiety of gp41 protein contains three essential functional regions: a fusion peptide (FP), a N-terminal heptad repeat (NHR) and a C-terminal heptad repeat (CHR). The NHR and CHR regions contain a number of leucine zipper-like motifs which have tendency to form coiled structures. *See* Peisajovich S, Shai Y, Biochem. Biophys. Acta 2003; 1614:122-129; Suarez T, et al., FEBS Lett. 2000; 477:145-149; Chan D, et al., Cell 1997; 89:263-273. The gp41 coiled structures shields several of its epitopes thus preventing the expression of antibodies against the protected antigens.

Due to their importance in the HIV replication cycle, many therapeutic strategies based on different forms of gp41 inhibition have been proposed. For instance, NHR and CHR derived peptides has been found to be effective in inhibiting HIV-1 fusion with the target cells. *See* Bolognesi D, et al., US 5,464,933; Bolognesi D, et al., US 6,133,418; Barney S, et al., US 6,093,794; Jiang S, et al., US 5,840,843. One CHR-peptide, enfuvirtide, was licensed in 2003 by the United States Food and Drug Administration as the first member of a new class of anti-HIV fusion inhibitor drugs.

However, one of the main limitations in the development of HIV vaccines is the design of immunogens able to elicit broad neutralizing antibodies. The design of these immunogens requires, firstly, the identification of conserved epitopes to assure a continuous and stable response, and secondly, devising new and more efficient immunogens that properly present those epitopes.

The use of neutralizing antibodies against of the envelope glycoprotein and its subunits has also been proposed to prevent HIV replication. *See* Yang X, et al., J. Virol. 2005; 79:3500-3508. Antibodies against gp41 epitopes or fusion proteins containing gp41 sequences have been described in the art. *See* Quinnan G, et al., WO2006/026508; Cherkasky A, W02006/136892.

However, there is still a need in the art for better immunogens that could induce the expression of specific antibodies against the gp41 protein and thus reduce the probability of a HIV infection.

### SUMMARY OF THE INVENTION

The present invention refers to different gp41 immunogens with enhanced immunogenic power. These immunogens are characterized by the removal of one or more immunodominant regions, like the HR1 and loop regions, of gp41. This approach has increased the suitability of the immunogens to lipidic environments and augmented their recognition by neutralizing antibodies.

In further aspects, the invention relates to nucleic acids encoding the gp41 variants, to vectors comprising said nucleic acids and to host cells comprising the nucleic acids and vectors indicated above.

In an additional aspect, the invention relates to vaccine compositions comprising the polypeptide, the nucleic acid sequences, the host cells comprising the nucleic acids and vectors of the inventions or combinations thereof. A further embodiment of the present invention refers to the use of these pharmaceutical compositions as medicaments.

In another aspect, the invention relates to a method for the detection of anti-gp41 antibodies in a sample which comprises contacting said sample with a polypeptide of the invention under conditions adequate for the formation of a complex between the antibodies in the sample and the polypeptide and detecting the formation of said complex.

### BRIEF DESCRIPTION OF THE DRAWINGS

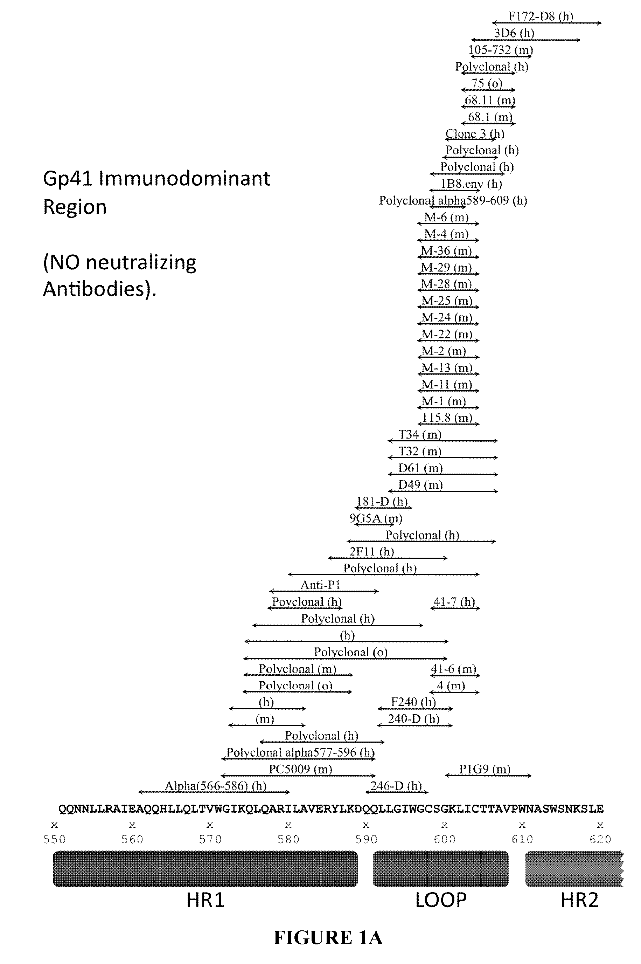

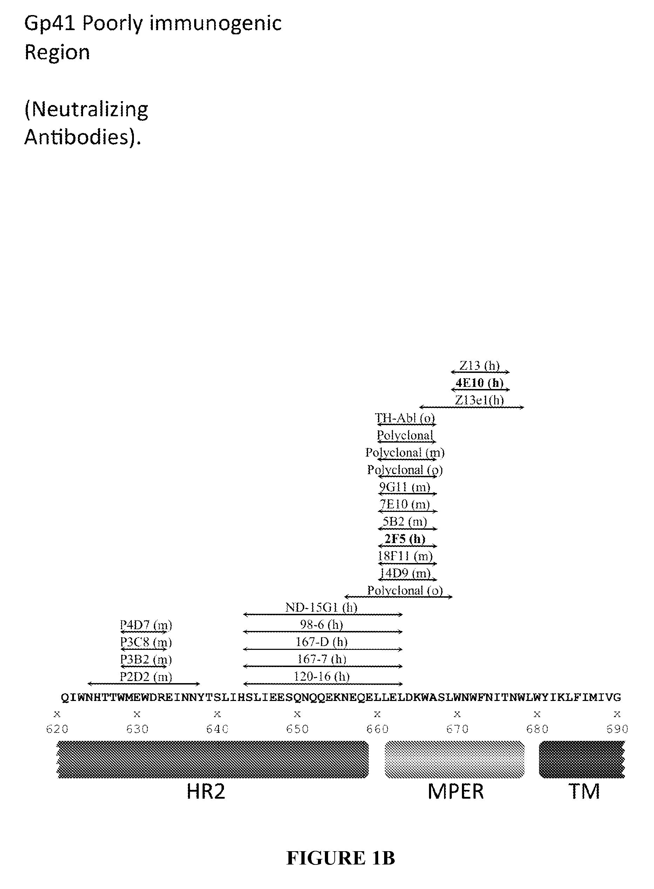

**Figure 1****.** Immune response to gp41 regions. The height of the columns, which show all identified antibodies, serves as a measure of immunogenicity. The different regions of gp41 are indicated in color code. **A:** immunodominant region. **B:** poorly immunogenic region.

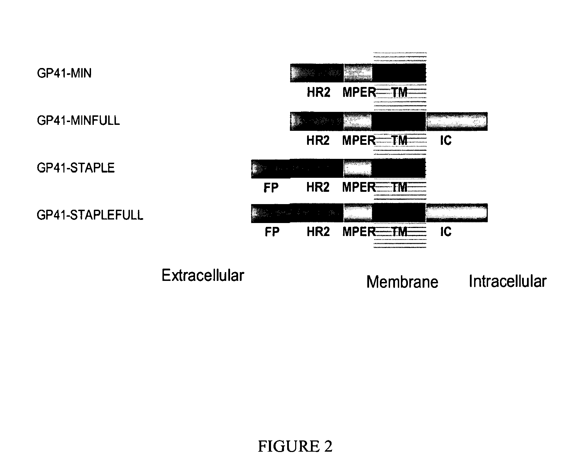

**Figure 2****.** Collection ofuntagged gp41 immunogens.
**Figure 3****.** Gp41 regions utilized in primer design. The regions are shown in a 5' to 3' sense and include overlapping sequences for the generation of fused proteins.
**Figure 4****.** GP41-MIN plasmid diagram and expected protein folding.
**Figure 5****.** GP41-MINFULL plasmid diagram and expected protein folding.
**Figure 6****.** GP41-STAPLE plasmid diagram and expected protein folding.
**Figure 7****.** GP41-STAPLEFULL plasmid diagram and expected protein folding.
**Figure 8****.** Surface expression of GP41-MIN immunogens in HEK-293T cells as tested by anti gp41 4E10 antibodies in a flow cytometry assay 48 hours after transfection.
**Figure 9****.** Surface expression of GP41-MIN immunogens in HEK-293T cells as tested by anti gp41 2F5 antibodies in a flow cytometry assay 48 hours after transfection.
**Figure 10****.** Surface expression of GP41-MINFULL immunogens in HEK-293T cells as tested by anti gp41 4E10 antibodies in a flow cytometry assay 48 hours after transfection.
**Figure 11****.** Surface expression of GP41-MINFULL immunogens in HEK-293T cells as tested by anti gp41 2F5 antibodies in a flow cytometry assay 48 hours after transfection.
**Figure 12****.** Surface expression of GP41-STAPLE immunogens in HEK-293T cells as tested by anti gp41 4E10 antibodies in a flow cytometry assay 48 hours after transfection.
**Figure 13****.** Surface expression of GP41-STAPLE immunogens in HEK-293T cells as tested by anti gp41 2F5 antibodies in a flow cytometry assay 48 hours after transfection.
**Figure 14****.** Surface expression of GP41-STAPLEFULL immunogens in HEK-293T cells as tested by anti gp41 4E10 antibodies in a flow cytometry assay 48 hours after transfection.
**Figure 15****.** Surface expression of GP41-STAPLEFULL immunogens in HEK-293T cells as tested by anti gp41 2F5 antibodies in a flow cytometry assay 48 hours after transfection.
**Figure 16****.** Final characterization of the gag HYGRO 293 T cell clone expressing only gag used for control purposes. The figure shows the cell-surface staining of the clone with anti gp41 2F5 antibodies.
**Figure 17****.** Final characterization of the gag HYGRO 293 T cell clone expressing only gag used for control purposes. The figure shows the intracellular staining of the clone with anti p24 Kc57 antibodies.
**Figure 18****.** Final characterization of the 293 T cell clone 4G2 2A expressing gag and the GP41-MINFULL immunogen. The figure shows the cell-surface staining of the clone with anti gp41 2F5 antibodies.
**Figure 19****.** Final characterization of the 293 T cell clone 4G2 2A expressing gag and the GP41-MINFULL immunogen. The figure shows the intracellular staining of the clone with anti p24 Kc57 antibodies.
**Figure 20****.** Final characterization of the 293 T cell clone 8G1 1D expressing gag and the GP41-STAPLEFULL immunogen. The figure shows the cell-surface staining of the clone with anti gp41 2F5 antibodies.
**Figure 21****.** Final characterization of the 293 T cell clone 8G1 1D expressing gag and the GP41-STAPLEFULL immunogen. The figure shows the intracellular staining of the clone with anti p24 Kc57 antibodies.
**Figure 22****.** VLP immunization protocol. The scale represent weeks.
**Figure 23****.** DNA vaccine immunization protocol. The scale represent weeks.

### DEPOSIT OF MICROORGANISMS

Vectors comprising the polynucleotides encoding the immunogens of the invention have been deposited as follows:
The plasmid pGP41-MINFULL was deposited on August 11^{th}, 2010, under access number DSM 23873 at the DSMZ - Deutsche Sammlung von Mikroorganismen und Zellkulturen, Inhoffenstraße 7 B, D-38124 Braunschweig, Federal Republic of Germany.
The plasmid pGP41-MIN was deposited on August 11^{th}, 2010, under access number DSM 23874 at the DSMZ - Deutsche Sammlung von Mikroorganismen und Zellkulturen, Inhoffenstraße 7 B, D-38124 Braunschweig, Federal Republic of Germany.
The plasmid pGP41-STAPLEFULL was deposited on August 11^{th}, 2010, under access number DSM 23875 at the DSMZ - Deutsche Sammlung von Mikroorganismen und Zellkulturen, Inhoffenstraße 7 B, D-38124 Braunschweig, Federal Republic of Germany.
The plasmid pGP41-STAPLE was deposited on August 11^{th}, 2010, under access number DSM 23876 at the DSMZ - Deutsche Sammlung von Mikroorganismen und Zellkulturen, Inhoffenstraße 7 B, D-38124 Braunschweig, Federal Republic of Germany.

### DETAILED DESCRIPTION OF THE INVENTION

The present invention demonstrates that by removing immunodominant regions in gp41 and presenting the gp41 MPER region in a more native lipidic environment a high immune response is obtained against the MPER region. This approach could be the basis for the production of neutralizing antibodies against a broad spectrum of HIV variants, as the MPER region is highly conserved.

Thus, in a first aspect, the invention relates to HIV gp41 polypeptide variants lacking at least one of the immunodominant regions in the extracellular domain.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, "HIV" is meant to include HIV-1 and HIV-2. "HIV- 1" means the human immunodeficiency virus type-1. HIV-1 includes but is not limited to extracellular virus particles and the forms of HIV-1 associated with HIV-1 infected cells. "HIV-2" means the human immunodeficiency virus type-2. HIV-2 includes but is not limited to extracellular virus particles and the forms of HIV-2 associated with HIV-2 infected cells.

The HIV-1 virus may represent any of the known major subtypes (Classes A, B, C, D E, F, G and H) or outlying subtype (Group O) including laboratory strains and primary isolates. The human immunodeficiency virus includes but is not limited to the JR-FL strain. "HIV-1_{JR-FL}" is a strain that was originally isolated from the brain tissue of an AIDS patient taken at autopsy and co-cultured with lectin-activated normal human PBMCs. *See* O'Brien W, et al., Nature 1990; 348:69-73. HIV-1_{JR-FL} is known to utilize CCR5 as a fusion co-receptor and has the ability to replicate in phytohemagglutinin (PHA) -stimulated PBMCs and blood-derived macrophages but does not replicate efficiently in most immortalized T cell lines. The gp41 may also be derived from other commonly used HIV-1 strains such as HIV-1_{DH123}, HIV-1_{Gun-1}, HIV-1₈₉.₆, and HIV-1_{HXB2}.

The term "gp41 ", as used herein, refers to human immunodeficiency virus-1 envelope glycoprotein gp41. Gp41 is a subunit, which forms the Env glycoprotein of HIV-1 together with gp120. Env is a trimer composed of three external subunits (gp120) and three transmembrane subunits (gp41). The nucleic acid and amino acid sequences of a large number of HIV gp-41 are readily available to the public. *See* HIV Sequence Database, http://www.hiv.lanl.gov/content/sequence/HIV/mainpage.html, August 20010; Los Alamos HIV Databases and Compendia, http://www.hiv.lanl.gov/content/sequence/HIV/refer.html, August 2010.

The term "immunodominant region", as used herein, refers to a region (i.e. segments or portions) of a protein from a pathogenic organism that, when administered to a mammal, are capable of inducing a protective immune response to the organism in the mammal. The immunodominant region may comprise one or more epitopes, Generally, but not always, such immunodominant regions or epitopes are highly immunogenic when tested according to methods that are known to those of skill in the art, such as those described herein. In the particular case of gp41, immunodominant regions are located at amino acid positions 584-618 and, more particularly, at positions 598-609. *See* Shin S, et al., Biochem. Mol. Biol. Intl. 1997; 43(4):713-721; Gnann J, et al., J. Virol. 1987; 61:2639-2641.

The "extracellular domain" or "ectodomain" or HIV-1 gp41 is meant to refer to the part of the protein that is on the outside of the cell. In certain examples, the extracellular domain of gp41 consists of the region between positions 512 and 683 according to HXB2 numbering.

Testing the immunogenicity of different forms of the gp41 ectodomain in which the fusion peptide, the HR1 or the loop have been sequentially deleted, we found that the exposure of the poorly immunogenic MPER sequence is increased when the following regions (that include the immunodominant loop) are removed. *See* Merat R, et al., J. Virol.1999; 73:5698-5706. Suitable regions that can be removed from HIBV gp41 in order to obtain the polypeptides of the invention are as follows:

| Epitope | Amino acids | HIV Isolate | SEQ ID NO: |
|---|---|---|---|
| AVGIGALFLGFLGAAGSTMGAASMTLTVQA | 512-531 | HXB2 | 1 |
| RQLLSGIVQQQNNLLRAIEAQQHLLQLTVWGIKQLQAR ILAVERYLKDQQLL | 532 to 592 | HXB2 | 2 |
| GIWGCSGKLICTTAVPWNASWSNKSLEQ | 593 to 621 | HXB2 | 3 |

However, the removal of the HR1 and the Loop sequences are sufficient to increase the exposure of the MPER epitope.

Because of sequence numbering variability among different HIV strains and isolates, it will be appreciated that the amino acid positions and the exact sequence of the immunodominant epitopes will not be the same in all HIV isolates. For example, in the case of the major gp41 immunodominant epitope identified above as SEQ ID NO:1, which is located at positions 591 to 620 in the HIV-1 85US_Ba-L isolate, the sequence appears at amino acids positions 593 to 622 in the HIV-1 HXB2 isolate encoded by the polynucleotide identified as accession No. K03455 in the NCBI database) and having the sequence: GIWGCSGKLICTTAVPWNASWSNKSLEQI (SEQ ID NO:4) and at amino acid positions 591 to 620 in the HIV-1 BRU isolate (accession number K02013 in the NCBI database) with the sequence:

GIWGCSGKLICTTAVPWNASWSNKSLEQI (SEQ ID NO:5)

It will be appreciated that once an immunodominant epitope is identified in a given gp41 sequence (or in the sequence of the gp120 precursor protein), the corresponding immunodominant epitope in the gp41 sequence of other HIV-1 isolates can be easily identified by aligning the sequence of both gp120 variants or by performing a multiple alignment of the gp41 wherein the epitope is to be identified within a plurality of other gp41 sequences.

For sequence comparison, typically one sequence acts as a reference, to which the test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. For sequence comparison of HIV envelope glycoproteins, fusion proteins comprising envelope glycoproteins and nucleic acid sequences encoding the same, the BLAST and BLAST 2.0 algorithms and the default parameters discussed below are used.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, for instance, by the Smith-Waterman local homology algorithm, by the Needleman-Wunsch homology alignment algorithm, by the Pearson-Lipman similarity search method, by computerized implementations of these algorithms or by manual alignment and visual inspection. *See* Smith T, Waterman M, Adv. Appl. Math. 1981; 2:482-489; Needleman S, Wunsch C, J. Mol. Biol. 1970; 48:443-453; Pearson W, Lipman D, Proc. Natl. Acad. Sci. USA 1988; 85:2444-2448; the GAP, BESTFIT, FASTA and TFASTA programs, Wisconsin Genetics Software Package, Genetics Computer Group, Madison, WI, USA; Ausubel F, et al., Eds" "Short Protocols in Molecular Biology", 4th Ed. (John Wiley and Sons, Inc., New York, NY, USA).

The BLAST and BLAST 2.0 algorithms are suitable for determining percent sequence identity and sequence similarity. *See* Altschul S, et al., Nuc. Acids Res. 1977; 25:3389-3402; Altschul S, et al., J. Mol. Biol. 1990; 215:403-410. The BLAST and BLAST 2.0 programs are used, with the parameters described herein, to determine percent sequence identity for the nucleic acids and proteins of the invention. Software for performing BLAST analyses is publicly available through the National Center for Biotechnology Information (http://blast.ncbi.nlm.nih.gov/blast.cgi, August 2010). This algorithm involves first identifying high scoring sequence pairs (HSPs) through the recognition of short words of length W in the query sequence, which either match or satisfy some positive-valued threshold score T when aligned with a word of the same length in a database sequence. T is referred to as the neighborhood word score threshold. *See* Altschul S, *et al*., 1997, 1990, *supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative- scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=4 and a comparison of both strands. For amino acid sequences, the BLASTP program uses as defaults a word length of 3, and expectation (E) of 10, and the BLOSUM62 scoring matrix alignments (B) of 50, expectation (E) of 10, M=5, N4, and a comparison of both strands. *See* Henikoff S, Henikoff J, Proc. Natl. Acad. Sci. USA 1989; 89:10915-10919. The BLAST algorithm also performs a statistical analysis of the similarity between two sequences. *See* Karlin S, Altschul S, Proc. Natl. Acad. Sci. USA 1993; 90:5873-5787. One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The "extracellular domain" or "ectodomain" or HIV-1 gp41 is meant to refer to the part of the protein that is the outside the cell. In certain examples, the extracellular domain of gp41 consists of position 512 to 683 of the gp160 precursor protein according to the numbering of the HXB2 env protein (shown in NCBI Protein database under accession number AAB50262 and depicted as SEQ ID NO:6).

1 MRVKEKYQHL WRWGWKWGTM LLGILMICSA TEKLWVTVYY GVPVWKEATT TLFCASDAKA
61 YDTEVHNVWA THACVPTDPN PQEVVLVNVT ENFNMWKNDM VEQMHEDIIS LWDQSLKPCV
121 KLTPLCVSLK CTDLGNATNT NSSNTNSSSG EMMMEKGEIK NCSFNISTSI RGKVQKEYAF
181 FYKLDIIPID NDTTSYTLTS CNTSVITQAC PKVSFEPIPI HYCAPAGFAI LKCNNKTFNG
241 TGPCTNVSTV QCTHGIRPVV STQLLLNGSL AEEEVVIRSA NFTDNAKTII VQLNQSVEIN
301 CTRPNNNTRK SIRIQRGPGR AFVTIGKIGN MRQAHCNISR AKWNATLKQI ASKLREQFGN
361 NKTIIFKQSS GGDPEIVTHS FNCGGEFFYC NSTQLFNSTW FNSTWSTEGS NNTEGSDTIT
421 LPCRIKQFIN MWQEVGKAMY APPISGQIRC SSNITGLLLT RDGGNNNNGS EIFRPGGGDM
481 RDNWRSELYK YKVVKIEPLG VAPTKAKRRV VQREKRAVGI GALFLGFLGA AGSTMGARSM
541 TLTVQARQLL SGIVQQQNNL LRAIEAQQHL LQLTVWGIKQ LQARILAVER YLKDQQLLGI
601 WGCSGKLICT TAVPWNASWS NKSLEQIWNN MTWMEWDREI NNYTSLIHSL IEESQNQQEK
661 NEQELLELDK WASLWNWFNI TNWLWYIKIF IMIVGGLVGL RIVFAVLSIV NRVRQGYSPL
721 SFQTHLPTPR GPDRPEGIEE EGGERDRDRS IRLVNGSLAL IWDDLRSLCL FSYHRLRDLL
781 LIVTRIVELL GRRGWEALKY WWNLLQYWSQ ELKNSAVSLL NATAIAVAEG TDRVIEVVQG
841 ACRAIRHIPR RIRQGLERIL L

Since the most conserved neutralization epitopes in gp41 are located in the HR1 region and in the loop region, the immunodominant epitopes in gp41 can be removed by deleting parts of or all of the HR1 and/or the loop region. Thus, in a preferred embodiment, the gp41 variant according to the invention lacks the HR1 region, preferably the complete HR1 region. In another preferred embodiment, the gp41 variant according to the invention lacks the loop region, preferably the complete loop region. In yet another preferred embodiment, the gp41 variant according to the invention lacks both the HR1 region and the loop region, preferably, both the complete HR1 and the loop regions.

As used herein, the terms "heptad repeat 1" and "HR1" are used indistinctly to refer to an heptad repeat region that is located at the amino terminus of wild-type gp41. A heptad repeat is a motif in which a hydrophobic amino acid is repeated every seven residues; such motifs are designated a through g. *See* Lupas A, Trends Biochem. Sci. 1996; 21:375-382. Heptad repeats which contain hydrophobic or neutral residues at the a and d positions can form alpha helices and are able to interact with other heptad repeats by forming coiled coils. *See* Chambers P, et al., J. Gen. Virol. 1990; 71:3075-3080; Lupas A, *supra.* The gp41 HR1 and HR2 sequences are well known in the art. *See* Miller M, et al., Proc. Natl. Acad. Sci. USA 2005; 102:14759-14764. In the particular case of the HXB2 env protein, the HR1 region corresponds to amino acids 542 to 591 of the polypeptide depicted in SEQ ID NO:6.

As used herein, the terms "loop region", "loop domain" or "C-C loop domain" are used indistinctly to refer to an immunodominant loop present in gp41 proteins that has a conserved disulfide bond. The HIV gp41 loop domain is well known in the art. In the particular case of the HXB2 env protein, the loop region corresponds to amino acids 592 to 621 of the polypeptide depicted in SEQ ID NO:6.

In a preferred embodiment, the gp41 variant according to the invention comprises, from the N- to C-terminal direction
(i) the heptad repeat 2 of HIV gp41 or an immunologically equivalent variant thereof,
(ii) the membrane proximal external region of HIV gp41 or an immunologically equivalent variant thereof and
(iii) the transmembrane region of HIV gp41 or an immunologically equivalent variant thereof.

In a still more preferred embodiment, the gp41 immunogen according to the invention is not the full-length gp41.

As used herein, the term "heptad repeat 2" refers to, but is not limited to, a heptad repeat region that is located at the carboxy terminus of wild-type gp41. The term "heptad repeat" has been defined above. In the particular case of the HXB2 env protein, the HR2 region corresponds to amino acids 623 to 664 of the polypeptide depicted in SEQ ID NO:6.

As used herein, the terms "membrane-proximal external region" and "MPER" refer, but are not limited to, a highly conserved region of the gp41 ectodomain adjacent to the viral membrane. In the particular case of the HXB2 env protein, the MPER region corresponds to amino acids 665 to 682 of the polypeptide depicted in SEQ ID NO:6.

As used herein, the term "transmembrane region" refers to the region of the gp41 polypeptide that is embedded into the plasma membrane once gp41 has acquired its native topology. In the particular case of the HXB2 env protein, the transmembrane region corresponds to amino acids 683 to 705 of the polypeptide depicted in SEQ ID NO:6.

In a preferred embodiment, the polypeptide of the invention further comprises the intracellular region of HIV gp41 or an immunologically equivalent variant thereof wherein the N-terminus of said intracellular region of HIV gp41 or of the immunologically equivalent variant thereof is connected to the C-terminus of the transmembrane region of HIV gp41 or the immunologically equivalent variant thereof.

The term "intracellular region" or "cytoplasmic tail" of HIV-1 gp 41 is meant to refer to the region corresponding to the portion of the protein that interacts with the interior of the cell or organelle. In certain examples, the cytoplasmic tail of gp41 corresponds to positions 706 to 856 of the polypeptide depicted in SEQ ID NO:6.

The numbering of the different gp41 regions is known in the art. *See* Yu H, et al., Retrovirol. 2008; 5:93 doi:10.1186/1742-4690-5-93.

In yet another preferred embodiment, the polypeptide of the invention further comprises the fusion peptide of HIV gp41 or an immunologically equivalent variant thereof wherein the C-terminus of the said fusion peptide or of the immunologically equivalent variant thereof is connected to the N-terminus of the heptad repeat 2 of HIV gp41 or of the functionally immunologically variant thereof.

The "fusion domain" of HIV-1 gp41 is meant to refer to the domain comprising the first 20 amino acids at the N-terminus of gp41. In certain examples, the fusion domain of gp-41 corresponds to positions 512-532 of the polypeptide depicted in SEQ ID NO:6.

As modifications and changes may be made in the structure of the regions forming the gp41 variants according to the invention and still obtain molecules having like or otherwise desirable characteristics, such immunologically functional equivalents are also encompassed within the scope of the present invention.

The term "immunologically functional equivalent" as used herein refers to a variant of a polypeptide that retains substantially equivalent ability to induce an immune response in a subject as the reference polypeptide. The term "immunologically functional equivalent" is well understood in the art and is further defined in detail herein. Immunologically functional equivalents may increase the antigenicity of a polypeptide, maintain the same level of antigenicity of the reference polypeptide, or decrease the antigenicity of a polypeptide only slightly so that it maintains its usefulness as an antigen in an immunogenic composition.

The term "substantially equivalent", as used herein, refers to variants which are able to generate an immune response which is differs from the immune response generated with the native region by no more than 5 %, no more than 10 %, no more than 15 %, no more than 20 %, no more than 25 %, no more than 30 %, no more than 35 %, no more than least 40 %, no more than 45 %, no more than 50 %, no more than 55 %, no more than 60 %, no more than 65 %, no more than 70 %, no more than 75 %, no more than 80 %, no more than 85 %, no more than 90 % or no more than 95 %.

For the purposes of the present invention, a polypeptide that is useful as an antigen in an immunogenic composition (i.e. has sufficient "immunogenic activity") can be identified by any method commonly known in the art for measuring the ability of a given polypeptide to trigger the generation of antibodies specific for said polypeptide when administered to a host organism. The ability of a given variant of the polypeptide of the invention to be an immunologically equivalent variant may be determined using standard neutralization assays, wherein the suspected variant is inoculated into a test animal and the resulting antibodies are tested for their ability to neutralize the infection of susceptible cells by HIV strains.

Any known and/or conventional method for screening and identifying and obtaining the neutralizing antibodies of the invention are contemplated by the present invention. In certain examples, a cell line or pseudovirus assay is used as a neutralization assay. Such assays are well known in the art.

In certain examples, a cell line/pseudovirus assay is used as a neutralization assay. Such assays are well-known in the art and easily performed by the skilled practicioner. Neutralizing antibody assays have been described as tools for assessing humoral immunity in AIDS virus infection and vaccine development. Suitable neutralization assays include, by way of example, the assays described by Montefiore (Curr. Protoc. Immunol., 2005 Jan;,Chapter 12:Unit 12.11.) utilizing a genetically engineered cell lines that are susceptible to infection by most strains of HIV-1, SIV, and SHIV. One assay is designed for optimal performance with uncloned viruses produced in either PBMC or CD4(+) T cell lines. A second assay is designed for single-cycle infection with molecularly cloned pseudoviruses produced by transfection in 293T cells. Both assays are performed in a 96-well format and use tat-responsive luciferase reporter gene expression as readout.

A safe and rapid neutralization assay using murine leukemia virus pseudotyped with HIV Type 1 envelope glycoprotein lacking the cytoplasmic domain has been disclosed. *See* Kim Y, et al., AIDS Res. Hum. Retroviruses. 2001; 17(18):1715-17249). In certain other examples, a peripheral blood mononuclear cells (PBMC) or primary isolates-based assay can be used as a neutralization assay. Such assays are well known in the art and could be performed easily by a skilled practitioner.

Montefiore et al. (J. Virol., 03 1997, 2512-2517, VoI 71, No. 3), incorporated by reference in its entirety herein, describe antibody-mediated neutralization of human immunodeficiency virus type 1 (HIV-I) with primary isolates and sera from infected individuals, using human peripheral blood mononuclear cells (PBMC). For the production of antibodies, various host animals may be immunized by injection with a protein, or a portion thereof (e.g. any one of the gp41 variants of the invention or the immunologically equivalent variants thereof). Such host animals may include but are not limited to rabbits, mice, and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.* Polyclonal antibodies are heterogeneous populations of antibody molecules derived from the sera of animals immunized with an antigen.

Immunologically functional equivalents of the gp41 regions according to the invention can be obtained by modifying the polypeptide. Said modifications can be, without limitation, amino acid changes, deletions, truncations, polypeptide fragments, fusions to other polypeptides, insertions, or any combination thereof. Accordingly, immunologically functional equivalents of the different gp41 regions show a degree of identity with respect to the amino acid sequence shown of the corresponding region in the native gp41 of at least 50%, at least 55%, at least 60%, at least 65%, at least 70%, at least 75%, at least 80%, at least 85%, at least 90%, at least 95%, at least 96%, at least 97%, at least 98% or at least 99% provided the immunogenic activity of the protein is maintained. Preferably, the identity exists over a region that is at least about 25 amino acids or nucleotides in length, or more preferably over a region that is 50-100 amino acids or nucleotides in length. An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. The degree of identity between two peptides can be determined using computer algorithms and methods, which are widely known by the persons skilled in the art. The identity between two amino acid sequences of two peptides is preferably determined using the BLASTP algorithm. *See* Altschul, S. et al., "BLAST Manual", (NCBI NLM NIH Bethesda, Md. 20894, Altschul, S., et al., J., 1990, Mol. Biol. 215:403-410).

As an example of modifications contemplated to be within the scope of the present invention, certain amino acids may be substituted for other amino acids in a polypeptide structure without appreciable loss of interactive binding capacity of the structure such as, for example, the epitope of an antigen that is recognized and bound by an antibody. Since it is the interactive capacity and nature of a polypeptide that defines its biological (e.g. immunological) functional activity, certain amino acid sequence substitutions can be made in an amino acid sequence (or its underlying DNA coding sequence) and nevertheless obtain a polypeptide with comparable properties. Various changes may be made to the amino acid sequences of the antigens of the present invention without appreciable loss of immunogenic activity.

It is understood in the art that in order to make functionally equivalent amino acid substitutions, the hydropathic index of amino acids may be considered. The importance of the hydropathic amino acid index in conferring interactive biological function on a polypeptide is generally understood in the art. *See* Kyte J, Doolittle R, J. Mol. Biol. 1982; 15(1):105-132. It is known that certain amino acids may be substituted for other amino acids having a similar hydropathic index or score and still retain a similar biological activity. In making changes based upon the hydropathic index, the substitution of amino acids whose hydropathic indices are within plus or minus 2 is preferred, those which are within plus or minus 1 are particularly preferred, and those within plus or minus 0.5 are even more particularly preferred. Each amino acid has been assigned a hydropathic index on the basis of its hydrophobicity and charge characteristics; these are: isoleucine (+4.5), valine (+4.2), leucine (+3.8), phenylalanine (+2.8), cysteine/cystine (+2.5), methionine (+1.9), alanine (+1.8), glycine (-0.4), threonine (-0.7), serine (-0.8), tryptophan (-0.9), tyrosine (-1.3), proline (-1.6), histidine (-3.2), glutamate (-3.5), glutamine (-3.5), aspartate (-3.5), asparagine (-3.5), lysine (-3.9) and arginine (-4.5).

It also is understood in the art that the substitution of similar amino acids can be made effectively on the basis of hydrophilicity; particularly where the immunologically functional equivalent polypeptide thereby created is intended for use in immunological embodiments, as in certain embodiments of the present invention. The greatest local average hydrophilicity of a protein, as governed by the hydrophilicity of its adjacent amino acids, correlates with its immunogenicity and antigenicity. *See* Hopp T, US 4,554,101. In making changes based upon similar hydrophilicity values, the substitution of amino acids whose hydrophilicity values are within plus or minus 2 is preferred, those which are within plus or minus 1 are particularly preferred, and those within plus or minus 0.5 are even more particularly preferred. The following hydrophilicity values have been assigned to amino acid residues: arginine (+3.0), lysine (+3.0), aspartate (+3.0 plus or minus 1), glutamate (+3.0 plus or minus 1), serine (+0.3), asparagine (+0.2), glutamine (+0.2), glycine (0), threonine (-0.4), proline (-0.5 plus or minus 1), alanine (-0.5), histidine (-0.5), cysteine (-1.0), methionine (-1.3), valine (-1.5), leucine (-1.8), isoleucine (-1.8), tyrosine (-2.3), phenylalanine (-2.5) and tryptophan (-3.4).

It is well known in the art that where certain residues are shown to be particularly important to the immunological or structural properties of a protein or peptide, like for example, residues in binding regions or epitopes, such residues may not generally be exchanged. In this manner, functional equivalents are defined herein as those polypeptides, which maintain a substantial amount of their native immunological activity. In general, the shorter the length of the molecule, the fewer changes can be made to the molecule without affecting its function. Longer domains may have an intermediate number of changes. The full-length protein will have the most tolerance for a larger number of changes. However, it must be appreciated that certain molecules or domains that are highly dependent upon their structure may tolerate little or no modification.

Immunologically equivalent variants can also be obtained by the expression from nucleic acid sequences which are substantially identical to the molecule encoding the regions in the native gp41 or their complements hybridize to each other under stringent conditions, as described below, provided that the immunogenic activity of the polypeptide encoded by said nucleic acids is preserved.

The phrase "stringent hybridization conditions" refers to conditions under which a probe will hybridize to its target subsequence, typically in a complex mixture of nucleic acid, but to no other sequences. Stringent conditions are sequence-dependent and will be different in different circumstances. Longer sequences hybridize specifically at higher temperatures. *See* Tijssen S, "Overview of principles of hybridization and the strategy of nucleic acid assays", Laboratory Techniques in Biochemistry and Molecular Biology (Elsevier Science Publishers B.V., Amsterdam, The Netherlands 1993). Generally, stringent conditions are selected to be about 5-10°C degrees lower than the thermal melting point (Tm) for the specific sequence at a defined ionic strength pH. The Tm is the temperature (under defined ionic strength, pH, and nucleic concentration) at which 50 percent of the probes complementary to the target hybridize to the target sequence at equilibrium (as the target sequences are present in excess, at Tm 50 percent of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g. 10 to 50 nucleotides) and at least about 60° C for long probes (e.g. greater than 50 nucleotides). Stringent conditions may also be achieved with the addition of destabilizing agents such as formamide. For high stringency hybridization, a positive signal is at least two times background, preferably 10 times background hybridization. Exemplary high stringency or stringent hybridization conditions include: 50 percent formamide, 5xSSC and 1 percent SDS incubated at 42°C or 5xSSC and 1 percent SDS incubated at 65°C, with a wash in 0.2xSSC and 0.1 percent SDS at 65°C.

Nucleic acids that do not hybridize to each other under stringent conditions are still substantially identical if the polypeptides that they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include an hybridization in a buffer of 40 percent formamide, 1 M NaCl, 1 percent SDS and 37°C. A positive hybridization is at least twice background. Those of ordinary skill in the art will readily recognize that alternative hybridization and wash conditions can be utilized to provide conditions of similar stringency. As used herein, the term "gp41-based HIV antigen" refers any antigen comprising gp41 or a fragment or derivative thereof.

A person skilled in the art will appreciate that the different regions which form part of the gp41 variants according to the present invention may be functionally linked directly or indirectly through a linker region. The term "linker region", as used herein, refers to any heterologous polypeptide of at least 1, 2, 3, 4 or 5 or more amino acids in length, which when inserted between a first and second region yields a functional linkage joining both regions and wherein each region is capable of preserving its functional and immunological properties.

In another preferred embodiment, the gp41 variants of the invention further comprise a tag which may be used for the detection or for the purification of the gp41 variant using reagents showing specific affinity towards said tags. Adequate detection/purification tags includes hexa-his (metal chelate moiety), hexa-hat GST (glutathione S-tranferase) glutathione affinity, calmodulin-binding peptide (CBP), strep-tag, cellulose binding domain, maltose binding protein, S-peptide tag, chitin binding tag, immuno-reactive epitopes, epitope tags, E2tag, HA epitope tag, Myc epitope, FLAG epitope, AU1 and AU5 epitopes, GIu-GIu epitope, KT3 epitope, IRS epitope, Btag epitope, protein kinase-C epitope, VSV epitope or any other tag as long as the tag does not affect the stability of the protein or the immunogenicity of the antigen attached thereto.

Moreover, different types of amino acid sequences may be added to the core gp41 immunogens to attain different objectives such as, for example, facilitating their handling or monitoring their expression. The addition of these peptide sequences to the core gp41 immunogens does not affect their immunogenic capabilities. For instance, a tetrad glycine encoding sequence was added downstream to the GP41-MINFULL and GP41-STAPLEFULL immunogen sequences as a linker to assist in their coupling to suitable vectors. A V5 epitope tag sequence was also added to the GP41-MINFULL and GP41-STAPLEFULL immunogen sequences to make possible their detection by Western blot analysis. Finally, a hexahistidine tag sequence was added to all the gp41 immunogen sequences of the present invention to facilitate their purification.

In a preferred embodiment, the gp41 variant comprises the gp41 HR2 region, the MPER region and the transmembrane region and lacks the fusion peptide, the HR1 region and the loop region. This variant is hereinafter referred to as GP41-MIN and has the sequence (SEQ ID NO:7):

1 MIWNNMTWME WDREINNYTS LIHSLIEESQ NQQEKNEQEL LELDKWASLW NWFNITNWLW
61 YIKLFIMIVG GLVGLRIVFA

In a preferred embodiment, the GP41-MIN immunogen contains a tetrad glycine linker and a V5 vector epitope tag at the C-terminus (SEQ ID NO: 8):

1 MIWNNMTWME WDREINNYTS LIHSLIEESQ NQQEKNEQEL LELDKWASLW NWFNITNWLW

61 YIKLFIMIVG GLVGLRIVFA VLSIVNRAGG GGKGQDNSAD IQHSGGRSSL EGPRFEGKPI

121 PNPLLGLDST RTG

In another preferred embodiment, the GP41-MIN immunogen contains an additional hexahistidine tag at the C-terminus (SEQ ID NO: 9):

1 MIWNNMTWME WDREINNYTS LIHSLIEESQ NQQEKNEQEL LELDKWASLW NWFNITNWLW

61 YIKLFIMIVG GLVGLRIVFA VLSIVNRAGG GGKGQDNSAD IQHSGGRSSL EGPRFEGKPI

121 PNPLLGLDST RTGHHHHHH

In another embodiment, the gp41 variant comprises the HR2 region, the MPER, the transmembrane domain and the intracellular domain and lacks all other gp41 regions (i.e. the fusion peptide, the HR1 region and the loop region). This variant (defined herein as GP41-MINFULL) has the sequence (SEQ ID NO:10):

1 MIWNNMTWME WDREINNYTS LIHSLIEESQ NQQEKNEQEL LELDKWASLW NWFNITNWLW
61 YIKLFIMIVG GLVGLRIVFA VLSIVNRVRQ GYSPLSFQTH LPTPRGPDRP EGIEEEGGER
121 DRDRSIRLVN GSLALIWDDL RSLCLFSYHR LRDLLLIVTR IVELLGRRGW EALKYWWNLL
181 QYWSQELKNS AVSLLNATAI AVAEGTDRVI EVVQGACRAI RHIPRRIRQG LERILL

In a preferred embodiment, the GP41-MINFULL immunogen contains a tetrad glycine linker and a V5 vector epitope tag at the C-terminus (SEQ ID NO: 11):

1 MIWNNMTWME WDREINNYTS LIHSLIEESQ NQQEKNEQEL LELDKWASLW NWFNITNWLW
61 YIKLFIMIVG GLVGLRIVFA VLSIVNRVRQ GYSPLSFQTH LPTPRGPDRP EGIEEEGGER
121 DRDRSIRLVN GSLALIWDDL RSLCLFSYHR LRDLLLIVTR IVELLGRRGW EALKYWWNLL
181 QYWSQELKNS AVSLLNATAI AVAEGTDRVI EVVQGACRAI RHIPRRIRQG LERILLGGGG
241 KGQDNSADIQ HSGGRSSLEG PRFEGKPIPN PLLGLDSTRT G

In another preferred embodiment, the GP41-MINFULL immunogen contains in addition a hexahistidine tag at the C-terminus (SEQ ID NO: 12):

1 MIWNNMTWME WDREINNYTS LIHSLIEESQ NQQEKNEQEL LELDKWASLW NWFNITNWLW
61 YIKLFIMIVG GLVGLRIVFA VLSIVNRVRQ GYSPLSFQTH LPTPRGPDRP EGIEEEGGER
121 DRDRSIRLVN GSLALIWDDL RSLCLFSYHR LRDLLLIVTR IVELLGRRGW EALKYWWNLL
181 QYWSQELKNS AVSLLNATAI AVAEGTDRVI EVVQGACRAI RHIPRRIRQG LERILLGGGG
241 KGQDNSADIQ HSGGRSSLEG PRFEGKPIPN PLLGLDSTRT GHHHHHH

In another embodiment, the gp41 variant of the invention comprises the fusion peptide, the HR2, the MPER and the transmembrane region and lacks all other gp41 regions (i.e. the HR1 region, the loop region and the intracellular region). This sequence is defined herein as GP41-STAPLE and has the sequence (SEQ ID NO: 13):

1 MAVGIGAMFL GFLGAAGSTM GAASMTLTVI WNHTTWMEWD REINNYTSLI HSLIEESQNQ
61 QEKNEQELLE LDKWASLWNW FNITNWLWYI KLFIMIVGGL VGLRIVFAVL SIVNRA

In a preferred embodiment, the GP41-MIN immunogen contains a tetrad glycine linker and a V5 vector epitope tag at the C-terminus (SEQ ID NO: 14):

1 MAVGIGAMFL GFLGAAGSTM GAASMTLTVI WNHTTWMEWD REINNYTSLI HSLIEESQNQ
61 QEKNEQELLE LDKWASLWNW FNITNWLWYI KLFIMIVGGL VGLRIVFAVL SIVNRAGGGG
121 KGQDNSADIQ HSGGRSSLEG PRFEGKPIPN PLLGLDSTRT G

In another preferred embodiment, the GP41-STAPLE immunogen contains an additional hexahistidine tag at the C-terminus (SEQ ID NO: 15):

1 MAVGIGAMFL GFLGAAGSTM GAASMTLTVI WNHTTWMEWD REINNYTSLI HSLIEESQNQ
61 QEKNEQELLE LDKWASLWNW FNITNWLWYI KLFIMIVGGL VGLRIVFAVL SIVNRAGGGG
121 KGQDNSADIQ HSGGRSSLEG PRFEGKPIPN PLLGLDSTRT GHHHHHH

In yet another embodiment, the gp41 variant comprises the fusion peptide, the HR2 region, the MPER, the transmembrane domain and the intracellular region and lacks all other gp41 regions (i.e. the HR1 region and the loop region). This variant is known as GP41-STAPLEFULL and shows the sequence (SEQ ID NO: 16):

1 MAVGIGAMFL GFLGAAGSTM GAASMTLTVI WNHTTWMEWD REINNYTSLI HSLIEESQNQ
61 QEKNEQELLE LDKWASLWNW FNITNWLWYI KLFIMIVGGL VGLRIVFAVL SIVNRVRQGY
121 SPLSFQTHLP TPRGPDRPEG IEEEGGERDR DRSIRLVNGS LALIWDDLRS LCLFSYHRLR
181 DLLLIVTRIV ELLGRRGWEA LKYWWNLLQY WSQELKNSAV SLLNATAIAV AEGTDRVIEV
241 VQGACRAIRH IPRRIRQGLE RILL

In a preferred embodiment, the GP41-STAPLEFULL immunogen contains a tetrad glycine linker and a V5 vector epitope tag at the C-terminus (SEQ ID NO: 17):

1   MAVGIGAMFL GFLGAAGSTM GAASMTLTVI WNHTTWMEWD REINNYTSLI HSLIEESQNQ
61  QEKNEQELLE LDKWASLWNW FNITNWLWYI KLFIMIVGGL VGLRIVFAVL SIVNRVRQGY
121 SPLSFQTHLP TPRGPDRPEG IEEEGGERDR DRSIRLVNGS LALIWDDLRS LCLFSYHRLR
181 DLLLIVTRIV ELLGRRGWEA LKYWWNLLQY WSQELKNSAV SLLNATAIAV AEGTDRVIEV
241 VQGACRAIRH IPRRIRQGLE RILLGGGGKG QDNSADIQHS GGRSSLEGPR FEGKPIPNPL
301 LGLDSTRTG

In another preferred embodiment, the GP41-STAPLEFULL immunogen contains in addition a hexahistidine tag at the C-terminus (SEQ ID NO: 18):

1 MAVGIGAMFL GFLGAAGSTM GAASMTLTVI WNHTTWMEWD REINNYTSLI HSLIEESQNQ
61 QEKNEQELLE LDKWASLWNW FNITNWLWYI KLFIMIVGGL VGLRIVFAVL SIVNRVRQGY
121 SPLSFQTHLP TPRGPDRPEG IEEEGGERDR DRSIRLVNGS LALIWDDLRS LCLFSYHRLR
181 DLLLIVTRIV ELLGRRGWEA LKYWWNLLQY WSQELKNSAV SLLNATAIAV AEGTDRVIEV
241 VQGACRAIRH IPRRIRQGLE RILLGGGGKG QDNSADIQHS GGRSSLEGPR FEGKPIPNPL
301 LGLDSTRTGH HHHHH

### Nucleic acids, vectors, host cells of the invention

The polypeptide also provides polynucleotides encoding the gp41 variants according to the invention.

A "nucleic acid," "polynucleotide," or "nucleic acid molecule" is a polymeric compound comprised of covalently linked subunits called nucleotides. Nucleic acid includes ribonucleic acid (RNA) and deoxyribonucleic acid (DNA), both of which may be single-stranded or double-stranded. DNA includes cDNA, genomic DNA, synthetic DNA, and semi-synthetic DNA. In a preferred embodiment, the nucleic acid of the invention encodes GP41-MIN and shows the sequence (SEQ ID NO:19): 
1 ATGATTTGGA ATAACATGAC CTGGATGGAG TGGGACAGAG AAATTAACAA TTACACAAGC

61 TTAATACACT CCTTAATTGA AGAATCGCAA AACCAGCAAG AAAAGAATGA ACAAGAATTA
 
121 TTGGAATTAG ATAAATGGGC AAGTTTGTGG AATTGGTTTA ACATAACAAA TTGGCTGTGG
181 TACATAAAAT TATTCATAAT GATAGTAGGA GGCTTGGTAG GTTTAAGAAT AGTTTTTGCT
241 GTACTTTCTA TAGTGAATAG AGCTGGTGGC GGTGGC
 or the variant thereof comprising an additional 123 nucleotide stretch at the 3' terminal which encodes a tetrad glycine linker and a V5 vector epitope tag having the sequence (SEQ ID NO:20): 
1 ATGATTTGGA ATAACATGAC CTGGATGGAG TGGGACAGAG AAATTAACAA TTACACAAGC
61 TTAATACACT CCTTAATTGA AGAATCGCAA AACCAGCAAG AAAAGAATGA ACAAGAATTA
121 TTGGAATTAG ATAAATGGGC AAGTTTGTGG AATTGGTTTA ACATAACAAA TTGGCTGTGG
181 TACATAAAAT TATTCATAAT GATAGTAGGA GGCTTGGTAG GTTTAAGAAT AGTTTTTGCT
241 GTACTTTCTA TAGTGAATAG AGCTGGTGGC GGTGGCAAGG GTCAAGACAA TTCTGCAGAT
301 ATCCAGCACA GTGGCGGCCG CTCGAGTCTA GAGGGCCCGC GGTTCGAAGG TAAGCCTATC
361 CCTAACCCTC TCCTCGGTCT CGATTCTACG CGTACCGGT
 or the variant thereof comprising an additional 18 nucleotide stretch at the 3' terminal which encodes a hexahistidine tag having the sequence (SEQ ID NO:21):

1 ATGATTTGGA ATAACATGAC CTGGATGGAG TGGGACAGAG AAATTAACAA TTACACAAGC
61 TTAATACACT CCTTAATTGA AGAATCGCAA AACCAGCAAG AAAAGAATGA ACAAGAATTA
121 TTGGAATTAG ATAAATGGGC AAGTTTGTGG AATTGGTTTA ACATAACAAA TTGGCTGTGG
181 TACATAAAAT TATTCATAAT GATAGTAGGA GGCTTGGTAG GTTTAAGAAT AGTTTTTGCT
241 GTACTTTCTA TAGTGAATAG AGCTGGTGGC GGTGGCAAGG GTCAAGACAA TTCTGCAGAT
301 ATCCAGCACA GTGGCGGCCG CTCGAGTCTA GAGGGCCCGC GGTTCGAAGG TAAGCCTATC
361 CCTAACCCTC TCCTCGGTCT CGATTCTACG CGTACCGGTC ATCATCACCA TCACCAT

In another preferred embodiment, the nucleic acid of the invention encodes GP41-MINFULL core which comprises the sequence (SEQ ID NO:22):: 
1 ATGATTTGGA ATAACATGAC CTGGATGGAG TGGGACAGAG AAATTAACAA TTACACAAGC
61 TTAATACACT CCTTAATTGA AGAATCGCAA AACCAGCAAG AAAAGAATGA ACAAGAATTA
121 TTGGAATTAG ATAAATGGGC AAGTTTGTGG AATTGGTTTA ACATAACAAA TTGGCTGTGG
181 TATATAAAAT TATTCATAAT GATAGTAGGA GGCTTGGTAG GTTTAAGAAT AGTTTTTGCT
241 GTACTTTCTA TAGTGAATAG AGTTAGGCAG GGATATTCAC CATTATCGTT TCAGACCCAC
301 CTCCCAACCC CGAGGGGACC CGACAGGCCC GAAGGAATAG AAGAAGAAGG TGGAGAGAGA
361 GACAGAGACA GATCCATTCG ATTAGTGAAC GGATCCTTGG CACTTATCTG GGACGATCTG
421 CGGAGCCTGT GCCTCTTCAG CTACCACCGC TTGAGAGACT TACTCTTGAT TGTAACGAGG
481 ATTGTGGAAC TTCTGGGACG CAGGGGGTGG GAAGCCCTCA AATATTGGTG GAATCTCCTA
541 CAGTATTGGA GTCAGGAACT AAAGAATAGT GCTGTTAGCT TGCTCAATGC CACAGCCATA
601 GCAGTAGCTG AGGGGACAGA TAGGGTTATA GAAGTAGTAC AAGGAGCTTG TAGAGCTATT
 661 CGCCACATAC CTAGAAGAAT AAGACAGGGC TTGGAAAGGA TTTTGCTA or the variant thereof comprising an additional 123 nucleotide stretch at the 3' terminal which encodes a tetrad glycine linker and a V5 vector epitope tag having the sequence (SEQ ID NO:23): 
1 ATGATTTGGA ATAACATGAC CTGGATGGAG TGGGACAGAG AAATTAACAA TTACACAAGC
61 TTAATACACT CCTTAATTGA AGAATCGCAA AACCAGCAAG AAAAGAATGA ACAAGAATTA
121 TTGGAATTAG ATAAATGGGC AAGTTTGTGG AATTGGTTTA ACATAACAAA TTGGCTGTGG
181 TATATAAAAT TATTCATAAT GATAGTAGGA GGCTTGGTAG GTTTAAGAAT AGTTTTTGCT
241 GTACTTTCTA TAGTGAATAG AGTTAGGCAG GGATATTCAC CATTATCGTT TCAGACCCAC
301 CTCCCAACCC CGAGGGGACC CGACAGGCCC GAAGGAATAG AAGAAGAAGG TGGAGAGAGA
361 GACAGAGACA GATCCATTCG ATTAGTGAAC GGATCCTTGG CACTTATCTG GGACGATCTG
421 CGGAGCCTGT GCCTCTTCAG CTACCACCGC TTGAGAGACT TACTCTTGAT TGTAACGAGG
481 ATTGTGGAAC TTCTGGGACG CAGGGGGTGG GAAGCCCTCA AATATTGGTG GAATCTCCTA
541 CAGTATTGGA GTCAGGAACT AAAGAATAGT GCTGTTAGCT TGCTCAATGC CACAGCCATA
601 GCAGTAGCTG AGGGGACAGA TAGGGTTATA GAAGTAGTAC AAGGAGCTTG TAGAGCTATT
661 CGCCACATAC CTAGAAGAAT AAGACAGGGC TTGGAAAGGA TTTTGCTAGG TGGCGGTGGC
721 AAGGGTCAAG ACAATTCTGC AGATATCCAG CACAGTGGCG GCCGCTCGAG TCTAGAGGGC
781 CCGCGGTTCG AAGGTAAGCC TATCCCTAAC CCTCTCCTCG GTCTCGATTC TACGCGTACC
841 GGT
 or the variant thereof comprising an additional 18 nucleotide stretch at the 3' terminal which encodes a hexahistidine tag having the sequence (SEQ ID NO:24):

1 ATGATTTGGA ATAACATGAC CTGGATGGAG TGGGACAGAG AAATTAACAA TTACACAAGC
61 TTAATACACT CCTTAATTGA AGAATCGCAA AACCAGCAAG AAAAGAATGA ACAAGAATTA
121 TTGGAATTAG ATAAATGGGC AAGTTTGTGG AATTGGTTTA ACATAACAAA TTGGCTGTGG
181 TATATAAAAT TATTCATAAT GATAGTAGGA GGCTTGGTAG GTTTAAGAAT AGTTTTTGCT
241 GTACTTTCTA TAGTGAATAG AGTTAGGCAG GGATATTCAC CATTATCGTT TCAGACCCAC
301 CTCCCAACCC CGAGGGGACC CGACAGGCCC GAAGGAATAG AAGAAGAAGG TGGAGAGAGA
361 GACAGAGACA GATCCATTCG ATTAGTGAAC GGATCCTTGG CACTTATCTG GGACGATCTG
421 CGGAGCCTGT GCCTCTTCAG CTACCACCGC TTGAGAGACT TACTCTTGAT TGTAACGAGG
481 ATTGTGGAAC TTCTGGGACG CAGGGGGTGG GAAGCCCTCA AATATTGGTG GAATCTCCTA
541 CAGTATTGGA GTCAGGAACT AAAGAATAGT GCTGTTAGCT TGCTCAATGC CACAGCCATA
601 GCAGTAGCTG AGGGGACAGA TAGGGTTATA GAAGTAGTAC AAGGAGCTTG TAGAGCTATT
661 CGCCACATAC CTAGAAGAAT AAGACAGGGC TTGGAAAGGA TTTTGCTAGG TGGCGGTGGC
721 AAGGGTCAAG ACAATTCTGC AGATATCCAG CACAGTGGCG GCCGCTCGAG TCTAGAGGGC
781 CCGCGGTTCG AAGGTAAGCC TATCCCTAAC CCTCTCCTCG GTCTCGATTC TACGCGTACC
841 GGTCATCATC ACCATCACCA T

In another embodiment, the nucleic acid of the invention encodes the GP41-STAPLE variant and shows the sequence (SEQ ID NO:25): 
1 ATGGCAGTGG GAATAGGAGC TATGTTCCTT GGGTTCTTGG GAGCAGCAGG AAGCACTATG
61 GGCGCAGCCT CAATGACGCT GACGGTAATT TGGAATCACA CGACCTGGAT GGAGTGGGAC
121 AGAGAAATTA ACAATTACAC AAGCTTAATA CACTCCTTAA TTGAAGAATC GCAAAACCAG
 
181 CAAGAAAAGA ATGAACAAGA ATTATTGGAA TTAGATAAAT GGGCAAGTTT GTGGAATTGG
241 TTTAACATAA CAAATTGGCT GTGGTATATA AAATTATTCA TAATGATAGT AGGAGGCTTG
301 GTAGGTTTAA GAATAGTTTT TGCTGTACTT TCTATAGTGA ATAGAGCTGG TGGCGGTGGC
 or the variant thereof comprising an additional 123 nucleotide stretch at the 3' terminal which encodes a tetrad glycine linker and a V5 vector epitope tag having the sequence (SEQ ID NO:26): 
1   ATGGCAGTGG GAATAGGAGC TATGTTCCTT GGGTTCTTGG GAGCAGCAGG AAGCACTATG
61  GGCGCAGCCT CAATGACGCT GACGGTAATT TGGAATCACA CGACCTGGAT GGAGTGGGAC
121 AGAGAAATTA ACAATTACAC AAGCTTAATA CACTCCTTAA TTGAAGAATC GCAAAACCAG
181 CAAGAAAAGA ATGAACAAGA ATTATTGGAA TTAGATAAAT GGGCAAGTTT GTGGAATTGG
241 TTTAACATAA CAAATTGGCT GTGGTATATA AAATTATTCA TAATGATAGT AGGAGGCTTG
301 GTAGGTTTAA GAATAGTTTT TGCTGTACTT TCTATAGTGA ATAGAGCTGG TGGCGGTGGC
361 AAGGGTCAAG ACAATTCTGC AGATATCCAG CACAGTGGCG GCCGCTCGAG TCTAGAGGGC
421 CCGCGGTTCG AAGGTAAGCC TATCCCTAAC CCTCTCCTCG GTCTCGATTC TACGCGTACC
481 GGT
 or the variant thereof comprising an additional 18 nucleotide stretch at the 3' terminal which encodes a hexahistidine tag having the sequence (SEQ ID NO:27):

1 ATGGCAGTGG GAATAGGAGC TATGTTCCTT GGGTTCTTGG GAGCAGCAGG AAGCACTATG
61 GGCGCAGCCT CAATGACGCT GACGGTAATT TGGAATCACA CGACCTGGAT GGAGTGGGAC
121 AGAGAAATTA ACAATTACAC AAGCTTAATA CACTCCTTAA TTGAAGAATC GCAAAACCAG
181 CAAGAAAAGA ATGAACAAGA ATTATTGGAA TTAGATAAAT GGGCAAGTTT GTGGAATTGG
241 TTTAACATAA CAAATTGGCT GTGGTATATA AAATTATTCA TAATGATAGT AGGAGGCTTG
301 GTAGGTTTAA GAATAGTTTT TGCTGTACTT TCTATAGTGA ATAGAGCTGG TGGCGGTGGC
361 AAGGGTCAAG ACAATTCTGC AGATATCCAG CACAGTGGCG GCCGCTCGAG TCTAGAGGGC
421 CCGCGGTTCG AAGGTAAGCC TATCCCTAAC CCTCTCCTCG GTCTCGATTC TACGCGTACC
481 GGTCATCATC ACCATCACCA T

In yet another embodiment, the polynucleotide encodes the GP41-STAPLEFULL core variant and shows the sequence (SEQ ID NO:28):: 
1   ATGGCAGTGG GAATAGGAGC TATGTTCCTT GGGTTCTTGG GAGCAGCAGG AAGCACTATG
 61   GGCGCAGCCT CAATGACGCT GACGGTAATT TGGAATCACA CGACCTGGAT GGAGTGGGAC
121   AGAGAAATTA ACAATTACAC AAGCTTAATA CACTCCTTAA TTGAAGAATC GCAAAACCAG
181   CAAGAAAAGA ATGAACAAGA ATTATTGGAA TTAGATAAAT GGGCAAGTTT GTGGAATTGG
241   TTTAACATAA CAAATTGGCT GTGGTATATA AAATTATTCA TAATGATAGT AGGAGGCTTG
301   GTAGGTTTAA GAATAGTTTT TGCTGTACTT TCTATAGTGA ATAGAGTTAG GCAGGGATAT
361   TCACCATTAT CGTTTCAGAC CCACCTCCCA ACCCCGAGGG GACCCGACAG GCCCGAAGGA
421   ATAGAAGAAG AAGGTGGAGA GAGAGACAGA GACAGATCCA TTCGATTAGT GAACGGATCC
481   TTGGCACTTA TCTGGGACGA TCTGCGGAGC CTGTGCCTCT TCAGCTACCA CCGCTTGAGA
541   GACTTACTCT TGATTGTAAC GAGGATTGTG GAACTTCTGG GACGCAGGGG GTGGGAAGCC
601   CTCAAATATT GGTGGAATCT CCTACAGTAT TGGAGTCAGG AACTAAAGAA TAGTGCTGTT
661   AGCTTGCTCA ATGCCACAGC CATAGCAGTA GCTGAGGGGA CAGATAGGGT TATAGAAGTA
721   GTACAAGGAG CTTGTAGAGC TATTCGCCAC ATACCTAGAA GAATAAGACA GGGCTTGGAA
781   AGGATTTTGC TA
 or the variant thereof comprising an additional 123 nucleotide stretch at the 3' terminal which encodes a tetrad glycine linker and a V5 vector epitope tag having the sequence (SEQ ID NO:29): 
1 ATGGCAGTGG GAATAGGAGC TATGTTCCTT GGGTTCTTGG GAGCAGCAGG AAGCACTATG
61 GGCGCAGCCT CAATGACGCT GACGGTAATT TGGAATCACA CGACCTGGAT GGAGTGGGAC
121 AGAGAAATTA ACAATTACAC AAGCTTAATA CACTCCTTAA TTGAAGAATC GCAAAACCAG
181 CAAGAAAAGA ATGAACAAGA ATTATTGGAA TTAGATAAAT GGGCAAGTTT GTGGAATTGG
241 TTTAACATAA CAAATTGGCT GTGGTATATA AAATTATTCA TAATGATAGT AGGAGGCTTG
301 GTAGGTTTAA GAATAGTTTT TGCTGTACTT TCTATAGTGA ATAGAGTTAG GCAGGGATAT
361 TCACCATTAT CGTTTCAGAC CCACCTCCCA ACCCCGAGGG GACCCGACAG GCCCGAAGGA
421 ATAGAAGAAG AAGGTGGAGA GAGAGACAGA GACAGATCCA TTCGATTAGT GAACGGATCC
481 TTGGCACTTA TCTGGGACGA TCTGCGGAGC CTGTGCCTCT TCAGCTACCA CCGCTTGAGA
541 GACTTACTCT TGATTGTAAC GAGGATTGTG GAACTTCTGG GACGCAGGGG GTGGGAAGCC
601 CTCAAATATT GGTGGAATCT CCTACAGTAT TGGAGTCAGG AACTAAAGAA TAGTGCTGTT
661 AGCTTGCTCA ATGCCACAGC CATAGCAGTA GCTGAGGGGA CAGATAGGGT TATAGAAGTA
721 GTACAAGGAG CTTGTAGAGC TATTCGCCAC ATACCTAGAA GAATAAGACA GGGCTTGGAA
781 AGGATTTTGC TAGGTGGCGG TGGCAAGGGT CAAGACAATT CTGCAGATAT CCAGCACAGT
841 GGCGGCCGCT CGAGTCTAGA GGGCCCGCGG TTCGAAGGTA AGCCTATCCC TAACCCTCTC
901 CTCGGTCTCG ATTCTACGCG TACCGGT
 or the variant thereof comprising an additional 18 nucleotide stretch at the 3' terminal which encodes a hexahistidine tag having the sequence (SEQ ID NO:30):

1 ATGGCAGTGG GAATAGGAGC TATGTTCCTT GGGTTCTTGG GAGCAGCAGG AAGCACTATG
61 GGCGCAGCCT CAATGACGCT GACGGTAATT TGGAATCACA CGACCTGGAT GGAGTGGGAC
121 AGAGAAATTA ACAATTACAC AAGCTTAATA CACTCCTTAA TTGAAGAATC GCAAAACCAG
181 CAAGAAAAGA ATGAACAAGA ATTATTGGAA TTAGATAAAT GGGCAAGTTT GTGGAATTGG
241 TTTAACATAA CAAATTGGCT GTGGTATATA AAATTATTCA TAATGATAGT AGGAGGCTTG
301 GTAGGTTTAA GAATAGTTTT TGCTGTACTT TCTATAGTGA ATAGAGTTAG GCAGGGATAT
361 TCACCATTAT CGTTTCAGAC CCACCTCCCA ACCCCGAGGG GACCCGACAG GCCCGAAGGA
421 ATAGAAGAAG AAGGTGGAGA GAGAGACAGA GACAGATCCA TTCGATTAGT GAACGGATCC
481 TTGGCACTTA TCTGGGACGA TCTGCGGAGC CTGTGCCTCT TCAGCTACCA CCGCTTGAGA
541 GACTTACTCT TGATTGTAAC GAGGATTGTG GAACTTCTGG GACGCAGGGG GTGGGAAGCC
601 CTCAAATATT GGTGGAATCT CCTACAGTAT TGGAGTCAGG AACTAAAGAA TAGTGCTGTT
661 AGCTTGCTCA ATGCCACAGC CATAGCAGTA GCTGAGGGGA CAGATAGGGT TATAGAAGTA
721 GTACAAGGAG CTTGTAGAGC TATTCGCCAC ATACCTAGAA GAATAAGACA GGGCTTGGAA
781 AGGATTTTGC TAGGTGGCGG TGGCAAGGGT CAAGACAATT CTGCAGATAT CCAGCACAGT
841 GGCGGCCGCT CGAGTCTAGA GGGCCCGCGG TTCGAAGGTA AGCCTATCCC TAACCCTCTC
901 CTCGGTCTCG ATTCTACGCG TACCGGTCAT CATCACCATC ACCAT

The gp41 immunogen nucleotide sequences of the present invention may be obtained by several approaches. One method would be to remove the tag and linker sequences from the complete immunogen sequences to yield their intermediate and core sequences. This object could be attained by utilizing, for example, primers containing stop codons to end translation before the tag or linker nucleotide sequences are translated. Another method would be the addition of the tag and linker sequences to the core gp41 immunogens to yield their intermediate and full sequences. This object could be achieved, for example, by the use of restriction enzymes and ligases. A person having ordinary skill in the art would know how to perform the aforesaid additions or deletions to nucleotide sequences. *See* Brown T, 1995; Watson R, *et al.,* 1992; Alberts B, *et al.,* 2008; Innis M, *et al.,* 1990; Erlich H, 1989; Sambrook J, *et al.,* 1989 and Bishop T, *et al*., 1987, *infra.*

The nucleic acid molecule encoding the gp41 immunogens can be incorporated into a vector, for the purposes of cloning, performing other laboratory manipulations, manufacturing recombinant peptides or gene delivery.

A person skilled in the art will understand that there is no limitation as regards the type of vector that can be used. The vector can be a cloning vector, suitable for propagation and for obtaining polynucleotides, gene constructs or expression vectors incorporated to several heterologous organisms. Thus, suitable vectors according to the present invention include prokaryotic expression vectors (e.g. pUC 18, pUC 19, Bluescript and their derivatives), mp18, mp19, pBR322, pMB9, CoIE1, pCR1, RP4, phages and shuttle vectors (e.g. pSA3 and pAT28), yeast expression vectors (e.g. vectors of the type of 2 micron plasmids), integration plasmids, YEP vectors, centromeric plasmids and the like, insect cell expression vectors (e.g. the pAC series and pVL series vectors), plant expression vectors, such as vectors of expression in plants (e.g. pIBI, pEarleyGate, pAVA, pCAMBIA, pGSA, pGWB, pMDC, pMY, pORE series vectors), and eukaryotic expression vectors based on viral vectors (e.g. adenoviruses, viruses associated to adenoviruses as well as retroviruses and lentiviruses), as well as non-viral vectors such as pSilencer 4.1-CMV (Ambion, Applied Biosystems, Foster City, CA, USA), pcDNA3, pcDNA3.1/hyg pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

In a particular embodiments, the vector is an expression vector that can include a regulatory element, such as the cytomegalovirus hCMV immediate early gene, the early promoter of SV40 adenovirus, the late promoter of SV40 adenovirus, the lac system, the TAC system, the TRC system, the major operator and promoter regions of phage, the control regions of fd coat protein, the promoter for 3-phosphoglycerate kinase, the promoters of acid phosphatase or the promoters of the yeast mating factors. Exemplary vectors include bacterial artificial chromosomes, cosmids, yeast artificial chromosomes, phage, plasmids, lipid vectors and viral vectors (e.g. retrovirus). Particularly preferred are expression vectors which express the peptide of the invention as part of a fusion protein. Suitable fusion protein expression vectors have been described. *See* Kayman, et al., US 5,643,756. By the term "express", "expresses" or "expression" of a nucleic acid molecule it is meant that the sequence is transcribed, and optionally, translated. Typically, transcription and translation of a coding sequence will result in production of the gp41 immunogen according to the invention.

In another embodiment, the invention relates to a host cell which comprises the gp41 immunogens according to the invention, the nucleic acids encoding them or the vectors comprising the nucleic acids.

As used herein, the term "host cell" is intended to refer to a cell into which a nucleic acid of the invention, such as a recombinant expression vector of the invention, has been introduced. The terms "host cell" and "recombinant host cell" are used interchangeably herein. It should be understood that such terms refer not only to the particular subject cell but to the progeny, or potential progeny, of such a cell. Because certain modifications may occur in succeeding generations due to either mutation or environmental influences, such progeny may not, in fact, be identical to the parent cell, but can be still included within the scope of the term as used herein.

Cells suitable for the expression of the gp41 immunogens of the invention include, without limitation, mammal, plant, insect, fungal and bacterial cells. Bacterial cells include, without being limited to, Gram-positive bacterial cells such as species of the Bacillus, Streptomyces and Staphylococcus genus and Gram-negative bacterial cells such as cells of the Escherichia and Pseudomonas genus. Fungal cells preferably include cells of yeasts such as Saccharomyces, *Pichia pastoris* and *Hansenula polymorpha.* Insect cells include, without being limited to, Drosophila cells and Sf9 cells. Plant cells include, among others, cells of crop plants such as cereals, medicinal, ornamental or bulbous plants. Mammalian cells include, without limitation, Chinese hamster ovary (CHO) cells such as CHO-K1 (ATCC Accession No. CCL-61), DG44 (Chasin L, et al., 1986, Som. Cell Mol. Genet., 12:555-556; and Kolkekar A, et al., 1997, Biochemistry, 36:10901-10909), CHO-K1 Tet-On (Clontech, Mountain View, CA, USA), CHO designated ECACC 85050302 (CAMR, Salisbury, Wiltshire, UK), CHO clone 13 (GEIMG, Genova, IT), CHO clone B (GEIMG, Genova, IT), CHO-K1/SF designated ECACC 93061607 (CAMR, Salisbury, Wiltshire, UK), RR-CHOK1 designated ECACC 92052129 (CAMR, Salisbury, Wiltshire, UK), CHO cells negative for dihydrofolate reductase (CHO/-DHFR, Urlaub G, Chasin L, Proc. Natl. Acad. Sci. USA; 77:4216-4220), SV40-transformed monkey kidney CV1 cells (COS, COS-7, ATCC Accession No. CRL-1651); human embryonic kidney cells (293, 293T cells); baby hamster kidney cells (BHK, ATCC Accession No. CCL-IO); monkey kidney cells (CV1, ATCC Accession No. CCL-70); African Green Monkey kidney cells (VERO-76, ATCC Accession No. CRL-1587; VERO, ATCC Accession No. CCL-81); mouse Sertoli cells (TM4, Mather J, Biol. Reprod. 1980; 23:243-251); human cervical carcinoma cells (HELA, ATCC Accession No. CCL-2); dog kidney cells (MDCK, ATCC Accession No. CCL-34); human lung cells (W138, ATCC Accession No. CCL-75); human hepatoma cells (HEP-G2, HB 8065); mouse mammary tumor cells (MMT 060562, ATCC Accession No. CCL-51); buffalo rat liver cells (BRL 3A, ATCC Accession No. CRL- 1442); TRI cells (Mather J, Ann. NY Acad. Sci. 1982, 383:44-68); MCR 5 cells and FS4 cells. Preferably, the host cells utilized are HEK-293T cells.

In a preferred embodiment, the host cell of the invention is a recombinant bacterium. As used herein, the term "recombinant bacterium" refers to any bacterium that has been modified by the introduction of heterologous DNA. "Wild-type" or "control" bacterium include bacterium as found in its natural state without genetic manipulation or that are substantially identical to the recombinant bacterium, but do not express one or more of the proteins encoded by the heterologous DNA (e.g. contains a plasmid without the coding sequence of the heterologous polypeptide of interest). The term is intended to include progeny of the bacterium originally modified by the introduction of heterologous DNA. In a preferred embodiment, the bacterium is a gram negative bacterial cell, and this term is intended to include all facultative Gram-negative cells of the family Enterobacteriaceace such as Escherichia, Shigella, Citrobacter, Salmonella, Klebsiella, Enterobacter, Erwinia, Kluyvera, Serratia, Cedecea, Morganella, Hafhia, Edwardsiella, Providencia, Proteus and Yersinia.

### Virus-like particles of the invention

As used herein, the term "virus-like particle" or VLP refers a non-infectious biological construct designed to look like a virus. *See* St. Clair N, et al., Appl. Biol. Sci. 1999; 96:9469-9474; Granoff D, et al., W01998/050071. VLPs do not replicate because they do not include the genetic material (DNA or RNA) necessary for viral replication. The HIV gag proteins, like those of other retroviruses, are sufficient and necessary for the formation of VLPs. Retroviral gag proteins are generally synthesized as polyprotein precursors.

In a preferred embodiment, the GP41-MINFULL and GP41-STAPLEFULL immunogens are presented as VLPs. These VLPs are further characterized by containing an inner core formed by immature structural proteins (gag) enveloped by a lipid membrane bi-layer that exposes the appropriate immunogen on its surface. The immunogen is recruited at the sites of particle budding by the interaction of the gag precursor protein with the cytoplasmic tail of gp41.

### Vaccine formulations of the invention

The gp41 variants described herein are useful in vaccine compositions or compositions used to elicit a humoral or cellular immune response. Accordingly, the gp41 variant polypeptides and nucleic acids of the invention find application as a prophylactic vaccine, to reduce the replication of HIV-1 in already infected patients and limit the infectivity of virus in a vaccinated patient. Such vaccines can include a gp41 variant polypeptide, a nucleic acid molecule encoding the gp41 variant or a host cell expressing said gp41 immunogen. The gp41 immunogens could be further designed as a VLP. VLPs are well known adjuvants that can promote a vigorous immune response (i.e. B lymphocytes, helper T lymphocytes and cytotoxic T lymphocytes activation) against the appropriate immunogen.

When used in vaccine or immunogenic compositions, the gp41 immunogens of the present invention may be used as "subunit" vaccines or immunogens. Such vaccines or immunogens offer significant advantages over traditional vaccines in terms of safety and cost of production; however, subunit vaccines are often less immunogenic than whole-virus vaccines, and it is possible that adjuvants with significant immunostimulatory capabilities may be required in order to reach their full potential.

Currently, adjuvants approved for human use in the United States include aluminum salts (alum). These adjuvants have been useful for some vaccines including hepatitis B, diphtheria, polio, rabies, and influenza. Other useful adjuvants include Complete Freund's Adjuvant (CFA), Incomplete Freund's Adjuvant (IFA), muramyl dipeptide (MDP), synthetic analogues of MDP, N-acetylmuramyl-L-alanyl-D-isoglutamyl-L-alanine-2-[1,2-dipalmitoyl-s-glycero-3-
(hydroxyphosphoryloxy)]ethylamide (MTP-PE) and compositions containing a degradable oil and an emulsifying agent, wherein the oil and emulsifying agent are present in the form of an oil-in-water emulsion having oil droplets all of which are substantially less than one micron in diameter.

The vaccines of the invention may additionally comprise at least one adjuvant enhancing and/or mediating an immune response chosen from an innate immune response and/or an adaptative immune response. Usable adjuvants may enhance the immunological response by activating antigen presenting cells (APC), macrophages and/or stimulating specific sets of lymphocytes.

An adjuvant that may convene to the instant invention may be any ligand suitable for the activation of a pathogen recognition receptor (PRR) expressed in and on dendritic cells (DCs), T-cells, B-cells or other antigen presenting cells. Ligands activating the nucleotide-binding oligomerization domain (NOD) receptor pathway may be suited for the purpose of the invention. Adjuvants suitable for these ligands may be muramyl dipeptide derivatives. Ligands activating the toll-like receptors (TLRs) may also convene for the purpose of the invention. Those receptors are member of the PRR family and are widely expressed on a variety of innate immune cells, including DCs, macrophages, mast cells and neutrophils.

As example of ligands activating TLR, mention may be made, for TLR4 of monophosphoryl lipid A, 3-O-deacytylated monophosphoryl lipid A, LPS from *E. coli,* taxol, RSV fusion protein, and host heat shock proteins 60 and 70, for TLR2 of lipopeptides such as N-palmitoyl-S-2,3(bispalmitoyloxy)-propyl-cvsteinyl-seryl-(lysil)3-lysine, peptidoglycan of *S. aureus,* lipoproteins from *M. tuberculosis, S. cerevisiae* zymosan and highly purified *P. gingivalis* LPS; for TLR3 of dsRNA, TLR5 of flagellin and TLR7 synthetic compounds such as imidazoquinolines; or for TLR9 of certain types of CpG- rich DNA. Other useful adjuvants for the invention may be T helper epitopes.

A T helper epitope is a peptide usually derived from exogenous proteins that have undergone proteolytic degradation and processing within the endocytic pathway of antigen presenting cells (APCs). In those cells the major histocompatibility complex of class II (MHC II) associates with those peptides in endosomes. This complex transported to the surface of the APCs may interact with a specific T cell receptor of T lymphocytes CD4 leading to their activation. According to the helper epitope, the T cell response may be of Th1 and/or Th2 type, as known in the art.

An example of a Th-oriented response epitope is the pan DR helper T cell epitope (PADRE). This epitope is engineered to bind most common HLA-DR molecules with high affinity and to act as a powerful immunogen. The PADRE HTL epitope has been shown to augment the potency of vaccines designed to stimulate a cellular immune response. *See* Alexander J, et al., Immunol. Res. 1998; 18:79-92.

The formulation of vaccines or immunogenic compositions according to the invention will employ an effective amount of the antigen of interest. That is, it will include an amount of antigen, which in combination with the adjuvant will cause the subject to produce a specific and sufficient immunological response, so as to impart protection against a subsequent HIV to the subject. When used as an immunogenic composition, the formulation will contain an amount of antigen which, in combination with the adjuvant, will cause the subject to produce specific antibodies which may be used for diagnostic or therapeutic purposes.

The vaccine compositions of the invention may be useful for the prevention or therapy of HIV-1 infection. While all animals that can be afflicted with HIV-1 or their equivalents can be treated in this manner, the vaccines of the invention are directed particularly to their preventive and therapeutic uses in humans. Often, more than one administration may be required to bring about the desired prophylactic or therapeutic effect; the exact protocol (dosage and frequency) can be established by standard clinical procedures.

In accordance with the present invention, one or more gp41 variants according to the invention peptides can be combined with a suitable adjuvant (e.g. an aluminum salt) to create a vaccine. Vaccine formulations will contain an effective amount of the selected antigen(s) that when combined with an adjuvant will cause the vaccinated subject (e.g. chimpanzees, macaques, baboons or humans) to produce a sufficient and specific immunological response to the antigen. This response will promote the formation of neutralizing antibodies against subsequent HIV exposures. The vaccine compositions can also be used therapeutically for the treatment of subjects (e.g. chimpanzees, macaques, baboons or humans) already infected with HIV.

In various embodiments of the invention a pharmaceutically acceptable vehicle or carrier is an adjuvant; an aluminum salt or an oil-in-water emulsion; an emulsifying agent and a metabolizable oil; or an immunostimulating agent, and the composition is administered to the mammalian subject by injection. In some embodiments, it may be desirable to administer combination vaccines having one component that elicits an immune response primarily against macrophage-tropic HIV strains and a second component that elicits an immune response primarily against T cell-tropic HIV strains. It may also be desirable for either or both components to be composed of a mixture of antigens, as for example, a mixture of antigens each of which elicits an immune response to a particular HIV strain or group of HIV strains.

The invention also provides a method for stimulating the formation of antibodies capable of neutralizing infection by an HIV viral isolate in at least one mammalian species. Such a method includes immunizing a subject (i.e. a mammal) with a composition containing one or more gp41 variant polypeptides according to the invention so that antibodies capable of neutralizing infection by an HIV viral isolate are produced. Preferred peptides are those which can elicit antibodies which neutralize primary isolates in two or more clades (e.g. two or more of clades A, B, C, D and E). Antibody formation can be determined by obtaining a sample from the subject and evaluating whether the sample contains antibodies which specifically bind to the one or more gp41 variants peptides (e.g. by ELISA assays) and are capable of neutralizing one or more HIV viral isolates (e.g. in a neutralization assay).

Yet another object of the present invention is to provide expression systems that utilize nucleic acid vaccines. *See* André S, et al., J. Virol. 1998, 72:1497-1503; Mulligan M, Webber J, AIDS 1999; 13(Suppl A):S105-S112; O'Hagan D, et al., J. Virol. 2001; 75:9037-9043; Rainczuk A, et al., Infect. Immun. 2004; 72:5565-5573. The particular vector backbone of the nucleic acid vaccine in which sequences encoding Env3, gp 1403, Env, gp 140, or gp 120 and the M9 derivative are inserted is not important to the present invention and includes, but is not restricted to, pcDNA3.1ZEO and pVAX1 (Invitrogen, Carlsbad, CA, USA; Cat. Nos. V385-20 and V260-20, respectively); DNA sequences available at the Invitrogen website (http://www.invitrogen.com/site/us/en/home.html, August 2010); pNGVL (National Gene Vector Laboratory, University of Michigan, MI, USA); and p414cyc (ATCC Accession No. 87380) and p414GALS (ATCC Accession No. 87344).

The design, application, and immunology of DNA vaccines have been reviewed elsewhere. *See* Donnelly J, et al., Annu. Rev. Immunol. 1997; 15:617-648; Robinson H, Pertmer T, Adv. Virus Res. 2000; 55:1-74; Gurunathan S, et al., Annu. Rev. Immunol. 2000; 18:927-974 (2000); Ulmer J, Curr. Opin. Drug Discov. Devel. 2001; 4:192-197. Briefly, DNA vaccines are configured to direct the expression *in vivo* of a foreign gene (the immunogen) in the vaccine recipient and, consequently, to induce protection against a challenge with the pathogen. *See* Ulmer J, et al., Science 1993; 259:1745-1749; Robinson H, et al., Vaccine 1993; 11:957-960. Typically, the foreign gene is cloned into a bacterial plasmid that is optimized for expression in eukaryotes and consists of the following: (i) an origin of replication for propagation in bacteria; usually an *E. coli* origin such as ColE1 is used; (ii) an antibiotic resistance gene, usually kanamycin, for selection of the plasmid in bacteria; (iii) a strong promoter for optimal expression in mammalian cells like cytomegalovirus (CMV) or simian virus 40 (SV40) are typically used; (iv) multiple cloning site downstream of the promoter for insertion of the gene of interest and (v) SV40 or bovine growth hormone (BGH) polyadenylation signal for stabilization of mRNA.

Still another object of the present invention is to deliver vectors utilizing non-pathogenic or attenuated bacterial strains harboring plasmids capable of expressing the gp41 variants according to the invention, such as, but not restricted to *Escherichia spp., Salmonella spp., Shigella spp., Mycobacterium spp.* and *Listeria spp.*

The particular Escherichia strain employed is not critical to the present invention. Examples of Escherichia strains which can be employed in the present invention include *Escherichia coli* strains DH5a, HB 101, HS-4, 4608-58, 1184-68, 53638-C-17, 13-80, and 6-81, enterotoxigenic *E. coli,* enteropathogenic *E. coli* and enterohemorrhagic *E. coli. See* Sambrook *et al.,* infra; Sansonetti P, et al., Ann. Microbiol. 1982; 132A:351-355); Evans D, et al., Infect. Immun. 1975; 12:656-667; Donnenberg S, et al., J. Infect. Dis. 1994; 169:831-838; McKee M, O'Brien A, Infect. Immun. 1995; 63:2070-2074.

The particular Salmonella strain employed is not critical to the present invention. Examples of Salmonella strains that can be employed in the present invention include *S. typhi* (ATCC Accession No. 7251), *S. typhimurium* (ATCC Accession No. 13311), *S. galinarum* (ATCC Accession No. 9184), *S. enteriditis* (ATCC Accession No. 4931), *S. typhimurium* (ATCC Accession No. 6994) *S. typhi* aroC, aroD double mutant (Hone D, et al., Vaccine 1991; 9:810-816) and *S. typhimurium* aroA mutant (Mastroeni D, et al., Micro. Pathol. 1992; 13:477-491).

The particular Shigella strain employed is not critical to the present invention. Examples of Shigella strains that can be employed in the present invention include *S. flexneri* (ATCC Accession No. 29903), *S. flexneri* CVD1203 (ATCC Accession No. 55556), *S. flexneri* 15D (Sizemore D, et al., Vaccine 1997; 15:804-807; Sizemore D, et al., Science 1995, 270:299-302), *S. sonnei* (ATCC Accession No. 29930) and *S. dysenteriae* (ATCC Accession No. 13313).

The particular Mycobacterium strain employed is not critical to the present invention. Examples of Mycobacterium strains that could be employed in the present invention include *M. tuberculosis* CDC1551 strain (Griffith T, et al., Am. J. Respir. Crit. Care Med. 1995; 152:808-811), *M. tuberculosis* Beijing strain (van Soolingen D, et al., J. Clin. Microbiol. 1995; 33:3234-3238), *M. tuberculosis* H37Rv strain (ATCC Accession No. 25618), *M. tuberculosis* pantothenate auxotroph strain (Sambandamurthy V, Nat. Med. 2002; 8:1171-1174, *M. tuberculosis* rpoV mutant strain (Collins D, et al., Proc. Natl. Acad. Sci USA. 1995; 92: 8036, *M. tuberculosis* leucine auxotroph strain (Hondalus M, et al., Infect. Immun. 2000; 68(5):2888-2898), BCG Danish strain (ATCC Accession No. 35733), BCG Japanese strain (ATCC Accession No. 35737), BCG, Chicago strain (ATCC Accession No. 27289), BCG Copenhagen strain (ATCC No. 27290), BCG Pasteur strain (ATCC Accession No. 35734), BCG Glaxo strain (ATCC Accession No. 35741), BCG Connaught strain (ATCC Accession No. 35745) and BCG Montreal (ATCC Accession No. 35746).

The particular Listeria strain employed is not critical to the present invention. Examples of *Listeria monocytogenes* strains which can be employed in the present invention include, but are not restricted to *L. monocytogenes* strain 10403S (Stevens R, et al., J. Virol. 2004; 78:8210-8218), *L. ivanovii* and *L. seeligeri* strains (Haas A, et al., Biochim. Biophys. Acta. 1992; 1130:81-84) or mutant *L. monocytogenes* strains such as (i) actA plcB double mutant (Peters C, et al., FEMS Immunol. Med. Microbiol. 2003; 35:243-253; Angelakopoulos H, et al., Infect. Immun. 2002; 70:3592-3601); or (ii) dal dat double mutant for alanine racemase gene and D-amino acid aminotransferase gene (Thompson R, et al., Infect. Immun. 1998; 66:3552-3561).

Methods for delivering vectors using bacterial vehicles are well known in the art. *See* Hone D, et al., US 6,500,419; Powell R, et al., 5,877,159; Branstorm A, et al., US 5,824,538, Shata M, et al., Mol. Med. Today 2000; 6:66-71; Hone D, Shata M, J. Virol. 2001; 75:9665-9670; Shata M, et al., Vaccine 2001; 20:623-629; Rapp U and Kaufmann S, Int. Immunol. 2004, 16:597-605; Dietrich G, et al., Curr. Opin. Mol. Ther. 2003; 5:10-19; Gentschev I, et al., J. Biotechnol. 2000; 83:19-26. The type of plasmid delivered by said bacterial vehicles for expression of polynucleotides encoding said the gp41 variants of the invention is not important to the present invention and include plasmids with sequences encoding Env3, gp1403, Env, gp140, or gp120 and the M9 derivative inserted in expression vectors such as, but not restricted to, pcDNA3.1ZEO (Invitrogen, Carlsbad, CA, USA; Cat. No. V385-20); DNA sequence available at the Invitrogen website, *supra*), pNGVL (National Gene Vector Laboratory, University of Michigan, Ann Arbor, MI, USA), p414cyc (ATCC Accession No. 87380) and p414GALS (ATCC Accession No. 87344).

### Therapeutic methods of the invention

The terms "treat" and "treatment" refer to the therapeutic treatment wherein the subject has been diagnosed as having been infected by the HIV virus. The terms "treat" and "treatment" refer to preventing or slowing the infection and destruction of healthy CD4+ T cells in such a subject. It also refers to the prevention and slowing the onset of symptoms of the acquired immunodeficiency disease such as extreme low CD4+ T cell count and repeated infections by opportunistic pathogens such as *Mycobacteria spp., Pneumocystis carinii,* and *Pneumocystis cryptococcus.* Beneficial or desired clinical results include, but are not limited to, an increase in absolute naive CD4+ T-cell count (range 10-3520), an increase in the percentage of CD4+ T-cell over total circulating immune cells (range 1-50 percent), and/or an increase in CD4+ T-cell count as a percentage of normal CD4+ T-cell count in an uninfected subject (range 1-161 percent). "Treatment" can also mean prolonging survival of the infected subject as compared to expected survival if the subject did not receiving any HIV targeted treatment.

The vaccine compositions are administered in any conventional manner which will introduce the vaccine into the animal, usually by injection. For oral administration, the vaccine composition can be administered in a form similar to those used for the oral administration of other proteinaceous materials. As discussed above, the precise amounts and formulations for use in either prevention or therapy can vary depending on, for example, the circumstances of the inherent purity and activity of the antigen, any additional ingredients, carriers or the method of administration.

By way of non-limiting illustration, the vaccine dosages administered will typically be, with respect to the antigen, a minimum of about 0.1 mg/dose, more typically a minimum of about 1 mg/dose, and often a minimum of about 10 mg/dose. The maximum dosages are typically not as critical. Usually, however, the dosage will be no more than 500 mg/dose, often no more than 250 mg/dose. These dosages can be suspended in any appropriate pharmaceutical vehicle or carrier in sufficient volume to carry the dosage. Generally, the final volume, including carriers, adjuvants, and the like, typically will be at least 0.1 ml, more typically at least about 0.2 ml. The upper limit is governed by the practicality of the amount to be administered, generally no more than about 0.5 ml to about 1.0 ml.

In many cases the vaccine will need to be administered more than once to bring about the desired therapeutic or prophylactic effect. The precise protocol (dosage and frequency of administration can be established through standard clinical trials. Those skilled in the art will be able to design suitable clinical-trials using the results of animal trials (e.g. studies conducted in non-human primates). Dosages may range from 0.1 mg/dose to 1 mg/dose, 10 mg/dose, 100 mg/dose, or 250 mg/dose. The effective amount of a given peptide will depend on a number of factors including antigenicity and purity. In general, the antigen and adjuvant are suspended in a small volume (generally 2 ml or less) of a pharmaceutically acceptable carrier. Suitable adjuvants and vaccination protocols are described in the art. *See* Haigwood N, et al., US 5,614,612.

The terms "effective amount," or "effective dose" refers to the amount of an agent required to produce the intended pharmacological, therapeutic or preventive result. The pharmacologically effective amount results in the amelioration of one or more symptoms of a viral disorder, or prevents the advancement of a viral disease, or causes the regression of the disease or decreases viral transmission. For example, a therapeutically effective amount preferably refers to the amount of a therapeutic agent that decreases the rate of transmission, decreases HIV viral load, or decreases the number of infected cells, by at least 5 percent, preferably at least 10 percent, at least 15 percent, at least 20 percent, at least 25 percent, at least 30 percent, at least 35 percent, at least 40 percent, at least 45 percent, at least 50 percent, at least 55 percent, at least 60 percent, at least 65 percent, at least 70 percent, at least 75 percent, at least 80 percent, at least 85 percent, at least 90 percent, at least 95 percent, or more. A therapeutically effective amount, with reference to HIV, also refers to the amount of a therapeutic agent that increases CD4+ cell counts, increases time to progression to ADDS, or increases survival time by at least 5 percent, preferably at least 10 percent, at least 15 percent, at least 20 percent, at least 25 percent, at least 30 percent, at least 35 percent, at least 40 percent, at least 45 percent, at least 50 percent, at least 55 percent, at least 60 percent, at least 65 percent, at least 70 percent, at least 75 percent, at least 80 percent, at least 85 percent, at least 90 percent, at least 95 percent, or more.

The gp41 variants or vectors that express the said polypetide can be administered directly into animal tissues by intravenous, intramuscular, intradermal, intraperitoneal, intranasal and oral inoculation routes. The specific method used to introduce the gp41 immunogen variants or their expressing vectors into the target animal tissue is not critical to the present invention and can be selected from previously described vaccination procedures. *See* Wolff J, et al., Biotechniques 1991; 11:474-485; Johnston S, Tang D, Methods Cell Biol. 1994; 43:353-365; Yang N, Sun W, Nat. Med. 1995; 1:481-483; Qiu P, et al., Gene Ther. 1996; 3:262-268; Hone D, Shata M, 2001, *supra*; Shata M, 2001, *supra*; Buge S, et al., J. Virol. 1997; 71:8531-8541; Belyakov I, et al., Nat. Med. 2001; 7:1320-1326; Lambert J, et al., Vaccine 2001; 19:3033-3042; Kaneko H, et al., Virology 2000; 267:8-16; Belyakov I, et al., Proc. Natl. Acad. Sci. USA 1999; 96:4512-4517.

The immunogenicity of the gp41 variants or their expression vectors is assessed in an appropriate animal model (e.g. mice, rabbits, guinea pigs or Rhesus macaques). Initially the gp41 immunogen or its expressing vector is administered at a dosis appropriate to the formulation being used and are administered by an adequate oral, intranasal, subcutaneous or intramuscular route. The number of doses varies depending on the potency of the individual gp41 immunogen or the vectors expressing it and can be a single-, two-, three- or four-dose regimen spaced by 2- to 10-week intervals. Each immunogenicity study includes a negative control (e.g. ovalbumin or its expression vectors expressing it; keyhole limpet hemaglutinin (KLH) or its expression vectors) that does not contain or express a gp41 immunogen.

The efficacy of treatment of the invention can be assessed through different means such as, for example, by monitoring the viral load and CD4+ T cell count in the blood of an infected subject, by monitoring the serum IgG and IgA responses invoked by the gp41 immunogen or by measuring cellular immunity.

The monitoring of the viral load and CD4+ T cell count in the blood is carried out by standard procedures. If the vaccine is efficacious, there should be greater than or equal to one log reduction in viral load, preferably to less than 10,000 copies/ml HIV-RNA within 2-4 weeks after the commencement of treatment. If a reduction in viral load of less than 0.5 log is attained, or HIV-RNA stays above 100,000, then the treatment should be adjusted by either adding or switching drugs. Viral load measurement should be repeated every 4-6 months if the patient is clinically stable. If viral load returns to 0.3-0.5 log of pre-treatment levels, then the therapy is no longer working and should be changed. Within 2-4 weeks of starting treatment, CD4+ T-cell count should be increased by at least 30 cells/mm³. If this is not achieved, then the therapy should be changed. The CD4+ T-cell counts should be monitored every 3-6 months during periods of clinical stability, and more frequently, should symptomatic disease occur. If CD4+ T-cell count drops to baseline (or below 50 percent of increase from pre-treatment), then the therapy should be changed.

To measure serum IgG and IgA responses invoked by the gp41 immunogen, sera are collected 10, 20, 30, 40, 50, 60, 70, 80, 100, 200 and 365 days before and after vaccination. Blood is collected into individual tubes from the tail vein of each animal/human volunteer and allowed to clot by incubating for 4 hr on ice. After centrifugation in an appropriate size centrifuge (e.g. a microfuge for small samples and a Beckman Avanti 25i for large samples) for 5-30 min; the sera are transferred to fresh tubes and stored at -80°C. Mucosal IgG and IgA responses to antigens expressed by the genes of interest are determined using stools and vaginal washes that will be harvested before and regular intervals after vaccination. *See* Wu S, et al., Infect. Immun. 1995; 63:4933-4938; Wu S, et al., AIDS Res. Hum. Retrovir. 1997; 13:1187. Standard ELISAs are used to quantify the IgG and IgA responses to gp41 immunogens and native structures on HIV-1 Env in the sera and mucosal samples. *See* Abacioglu Y, et al., AIDS Res. Hum. Retrovir. 1994; 10:371-381. Ovalbumin can be included in each ELISA as a negative control antigen. In addition, each ELISA can include a positive control serum, stool or vaginal wash sample, as appropriate. The positive control samples are harvested from animals vaccinated intranasally with 10 µg of the gp41 immunogen mixed with 10 µg cholera toxin or sera harvested from HIV-infected individuals. *See* Bagley K, et al., Vaccine2003; 21:3335-3341. The end-point titers are calculated by taking the inverse of the last serum dilution that produced an increase in the absorbance at 490 nm that is greater than the mean of the negative control row plus three standard error values.

To measure cellular immunity, cell suspensions of enriched CD4+ and CD8+ T cells from lymphoid tissues are used to quantify antigen-specific T cell responses by cytokine-specific ELISPOT assay. *See* Wu S, *et al.,* 1995, 1997, *supra.* Such assays can measure the numbers of antigen-specific T cells that secrete IL-2, IL-4, IL-5, IL-6, IL-10 and IFN-gamma. All ELISPOT assays are conducted using commercially-available capture and detection mAbs (R&D Systems, Minneapolis, MN, USA; BD Biosciences Pharmingen, San Diego, CA, USA). *See* Wu S, *et al.,* 1995, 1997, *supra;* Shata M, 2001, *supra.* Each assay includes mitogen (Con A) and ovalbumin controls.

### Diagnostic assays of the invention

In addition to the use in vaccine formulations for the generation of anti-HIV antibodies and for the production of anti-HIV antiserum, the gp41 variants of the present invention may be used as diagnostic reagents in immunoassays to detect anti-HIV-1 antibodies. Many HIV-1 immunoassay formats are available. *See* Beltz G, et al., US 4,753,873; Paul D, et al., EP 0216191.

Immunoassay protocols may be based, for example, upon composition, direct reaction, or sandwich-type assays. Protocols may also, for example, be heterogeneous and use solid supports, or may be homogeneous and involve immune reactions in solution. Most assays involve the use of labeled antibodies or polypeptides. The labels may be, for example, fluorescent, chemiluminescent, radioactive or dye molecules. Assays which amplify the signals from the probe are also known, examples of such assays are those which utilize biotin and avidin, and enzyme-labeled and mediated immunoassays, such as ELISA assays.

Typically, an immunoassay for anti-HIV-1 antibody will involve selecting and preparing a test sample, such as a biological sample, and then incubating it with a gp41 immunogen of the present invention under conditions that allow antigen-antibody complexes to form. Such conditions are well known in the art. In a heterogeneous format, the protein or peptide is bound to a solid support to facilitate separation of the sample from the polypeptide after incubation. Examples of solid supports that can be used are nitrocellulose, in membrane or microtiter well form, polyvinylchloride, in sheets or microtiter wells, polystyrene latex, in beads or microtiter plates, polyvinlyidine fluoride, diazotized paper, nylon membranes, activated beads, and Protein A beads. Most preferably, Dynatech, Immulon(R) microtiter plates or 0.25 inch polystyrene beads are used in the heterogeneous format. The solid support is typically washed after separating it from the test sample.

In a homogeneous format, on the other hand, the test sample is incubated with the gp41 immunogen, under conditions that will precipitate any formed antigen-antibody complexes. The precipitated complexes are then separated from the sample through, for example, centrifugation. Depending on the format, the complexes can be detected with labeled anti-xenogenic immunoglobulin or, if a competitive format is used, by measuring the amount of bound, labeled competing antibody. These and other detection techniques and formats are well known in the art.

Diagnostic probes useful in the invention assays include antibodies to the HIV-1 envelope protein. The antibodies to may be either monoclonal or polyclonal produced using standard techniques well known in the art. *See* Harlow E, Lane D, "Antibodies: A Laboratory Manual" (Cold Spring Harbor Laboratory Press, Cold Spring Harbor, NY, USA, 1988). They can be used to detect HIV-1 envelope protein by specifically binding to the protein and subsequent detection of the antibody-protein complex by ELISA, Western blot or the like. The gp41 immunogens used to elicit these antibodies can be any of the variants discussed above. Antibodies are also produced from peptide sequences of modified gp41 immunogens using standard techniques in the art. *See* Harlow E, Lane D, *supra.* Fragments of monoclonal or polyclonal antisera containing the immunologically significant portion can also be prepared.

All publications mentioned hereinabove are hereby incorporated in their entirety by reference.

While the foregoing invention has been described in some detail for purposes of clarity and understanding, it will be appreciated by one skilled in the art from a reading of this disclosure that various changes in form and detail can be made without departing from the true scope of the invention and appended claims.

### EXAMPLES

### General Procedures

### 1. Primers

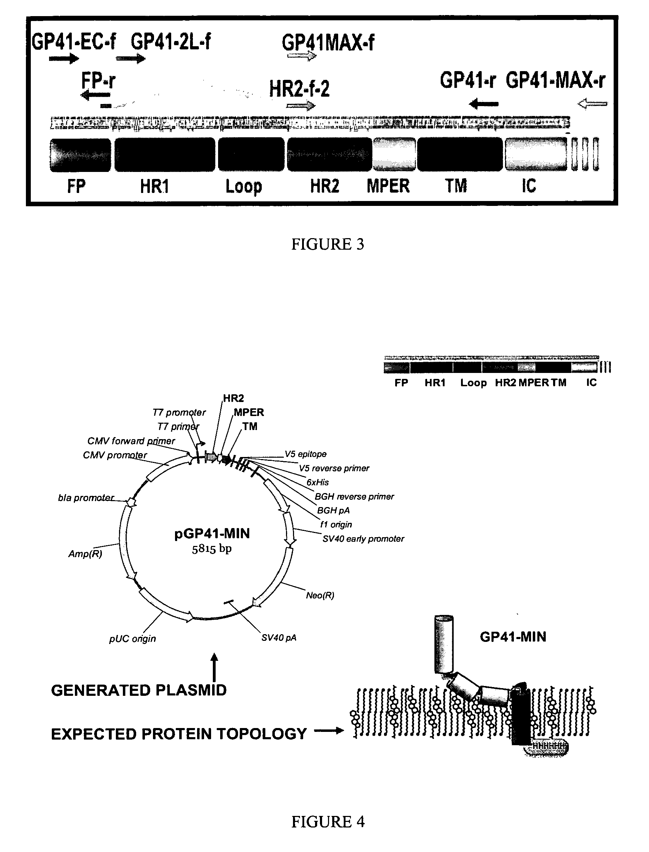

The primers were designed according to the scheme depicted in Figure 3. The sequences utilized for each region were as follows:

| Primer | Sequence | SEQ ID NO: |
|---|---|---|
| | *Fusion peptide region* | |
| GP41-EC-f | CACC**ATG**GCAGTGGGAATAGGAGCTATG | 31 |
| FP-r | TACCGTCAGCGTCATTGAGGCTG | 32 |
| | *HR1 region* | |
| GP41-2L-f | CACC**ATG**CAGGCCAGACAATTATTGTCTG | 33 |
| | *HR2 region* | |
| GP41-MAX-f | CACC**ATG**ATTTGGAATAACATGACCTGG | 34 |
| HR-2 f-2 | GCTGACGGTAATTTGGAATCACACGACCTGG | 35 |
| | *Transmembrane region* | |
| GP41-r | GCCACCGCCACCTAGCTCTATTCACTATAGA | 36 |
| | *Intracellular region* | |
| GP41-MAX-r | GCCACCGCCACCTAGCAAAATCCTTTCCA | 37 |

The underlined area indicates overhang sequences for directional cloning. The bold area indicates start/stop codons introduced into the sequence for expression. The doubled underlined area indicates overlapping sequences for the generation of fused proteins.

All amplification reactions were performed using Amplitaq DNA polymerase (Applied Biosystems, Foster City, CA, USA) in a 2720 Thermal cycler (Applied Biosystems, Foster City, CA, USA).

### 2. Templates

The pHXB2 plasmid containing a full HIV sequence and the peGFP plasmid containing an eGFP coding sequence were utilized.

### 3. Vectors

The final PCR products were cloned into a pCDNA vector using the pcDNA 3.1 Directional TOPO® Expression Kit (Invitrogen, Carlsbad, CA, USA; Cat. Nos. K4900-01, K4900-40). Stop codons were included in the designed gp41 primers to clone gp41 core sequences or not included to express the proteins carrying a V5 epitope and/or a 6xHis tag in their C terminus. These features facilitated their identification and purification, respectively.

For each immunogen, an additional plasmid containing the green fluorescent protein (GFP) coding sequence fused to the immunogen in C terminal was constructed. This plasmid served as tag for verifying expression of the related immunogen. In addition, the GFP-containing plasmid was also useful in the analysis of the immunogen expression pattern.

The PCR products were purified from nucleotides or primers and incubated with pcDNA3.1 open vectors for 10 minutes. The positive clones were selected in ampicillin plates, recovered, screened by PCR and then tested for functional expression. The plasmids were constructed and amplified according to molecular biology techniques well known in the art. *See* Brown T, "Gene Cloning" (Chapman & Hall, London, England, 1995); Watson R, et al., "Recombinant DNA", 2nd Ed. (Scientific American Books, New York, NY, USA, 1992); Alberts B, et al., "Molecular Biology of the Cell" (Garland Publishing Inc., New York, NY, USA, 2008); Innis M, et al., Eds., "PCR Protocols. A Guide to Methods and Applications" (Academic Press Inc., San Diego, CA, USA, 1990); Erlich H, Ed., "PCR Technology. Principles and Applications for DNA Amplification" (Stockton Press, New York, NY, USA, 1989); Sambrook J, et al., "Molecular Cloning. A Laboratory Manual" (Cold Spring Harbor Laboratory Press, Cold Spring Harbor, NY, USA, 1989); Bishop T, et al., "Nucleic Acid and Protein Sequence. A Practical Approach" (IRL Press, Oxford, England, 1987); Reznikoff W, Ed., "Maximizing Gene Expression" (Butterworths Publishers, Stoneham, MA, USA, 1987); Davis L, et al., "Basic Methods in Molecular Biology" (Elsevier Science Publishing Co., New York, NY, USA, 1986), Schleef M, Ed., "Plasmid for Therapy and Vaccination" (Wiley-VCH Verlag GmbH, Weinheim, Germany 2001).

### 4. Transient and stable expression in 293T cells and VLP production

### A. All immunogens

Human embryonic kidney HEK-293T cells (ATCC Accession No. CRL-11268) were cultured in DMEM medium (GIBCO®, Invitrogen, Carlsbad, CA, USA) supplemented with 10% fetal calf serum (GIBCO®, Invitrogen, Carlsbad, CA, USA) for 48 hours. One day before transfection, the cells were detached using versene (GIBCO®, Invitrogen, Carlsbad, CA, USA), washed in supplemented DMEM and split in six well plates at a density of 400000 cells/well. For transfection, each well was transfected with 2 µg of one of the plasmids coding for gp41 constructs described herein using the CalPhos Mammalian Transfection Kit (Clontech, Mountain View, CA, USA). Transient expression was assayed 24-48 hours after transfection.

Stable expression of gp41 proteins for large-scale immunogen production was assayed by flow cytometry after culturing transfected cells for two weeks in supplemented DMEM containing 1 mg/ml of the selection antibiotic G418 (GIBCO®, Invitrogen, Carlsbad, CA, USA). The transient and stable expressions were assayed by determining cell surface expression and total levels of gp41 proteins in cell extracts. In addition, molecular weight, western blot analysis, MPER integrity, antibody recognition and residual fusogenic activity assays were also performed.

### B. FULL immunogens

HEK-293T cells showing a high expression of the FULL (GP41-MINFULL and GP41-STAPLEFULL) immunogens were selected using a 1 cell per well sorting strategy. The individual cells were cultured in 96 well plates and screened for high expression of the immunogens. The cell clone of each immunogen showing the highest expression was expanded.

The selected clones were transfected with a plasmid coding for the gag sequence of HIV inserted in a PCDNA3.1 HYGRO vector (pCDNA3.1 gag-HYGRO vector). The cells were cultured in a DMEM medium (GIBCO®, Invitrogen, Carlsbad, CA, USA) supplemented with 10% fetal calf serum (GIBCO®, Invitrogen, Carlsbad, CA, USA) for 48 hours. The transfected cells were then cultured in a DMEM medium (GIBCO®, Invitrogen, Carlsbad, CA, USA) supplemented with hygromicin (0.1 mg/ml) for 2 weeks. The cells showing a high expression of the FULL immunogens and a high gag production were selected using a 1 cell per well cloning strategy. The individual cells were cultured in 96 well plates and screened for high expression of the gp41 immunogens and for the amount of gag in the supernatant (as a measure of VLP production). The cell clones showing the highest expression of the FULL immunogens and the gag protein were expanded.

### C. Generation of 293T cells stably producing nude VLPs

As a control for immunization, 293T cells were transfected with the PCDNA3.1 gag-HYGRO vector in a DMEM medium (GIBCO®, Invitrogen, Carlsbad, CA, USA) supplemented with 10% fetal calf serum (GIBCO®, Invitrogen, Carlsbad, CA, USA) for 48 hours. The transfected cells were then cultured in a DMEM medium (GIBCO®, Invitrogen, Carlsbad, CA, USA) supplemented with hygromicin (0.1 mg/ml) for 2 weeks to yield a population of 293T cells producing gag VLP stably without the FULL immunogens.

### D. VLP production

The control cells and the cells expressing the FULL immunogens were grown in serum-free medium 50% RPMI (GIBCO®, Invitrogen, Carlsbad, CA, USA) and 50% DMEN (GIBCO®, Invitrogen, Carlsbad, CA, USA) supplemented with 1% nutridoma-SP, 0.5mg/ml G418 and/or 0.05 mg/ml hygromicin-B. The supernatants were collected every 2-3 days, centrifuged 1000xg for 10 min, filtered through 0.45 µm filters and stored at -80°C.

The final VLP preparations were obtained in a two-step process from the culture supernatant as follows: i) thawed supernatant was concentrated in a 300kDa cut-off membrane that retains VLPs (10-30 fold) and ii) the retention material was further concentrated using 100 kDa cut-off membranes (10-30 fold). The final preparations (1-2 ml) were assayed for total protein content and for FULL immunogen and gag concentrations.

### 5. Molecular weight and Western blot analysis

Total levels and molecular weight of expressed proteins were assayed by Western blot. Briefly, cells (5 x 10⁶) were lysed with a 150 mM NaCl, 25 mM tris, 1 mM EDTA 1% Triton X-100 and 0.1% SDS buffer (pH 7.5). The lysates were stored at -80 °C until use. 10 µL of cell lysates were loaded onto 12% acrylamide NuPAGE Novex Bis-tris Mini gels (Invitrogen, Carlsbad, CA, USA) and electrophoresed in a X-cell Surelock machine (Invitrogen, Carlsbad, CA, USA). Gels were transferred to nitrocellulose membranes using an iBlot Dry blotting system (Invitrogen, Carlsbad, CA, USA).

Membranes were saturated in blocking buffer and then incubated overnight at 4 °C with the 2F5 anti-MPER monoclonal antibody (1 µg/ml). After washing excess of antibody, the membranes were revealed with IRDye 800 conjugated anti human IgG (Rockland Immunochemicals, Gilbertsville, PA, USA) and scanned in an Odyssey infrared imaging system (LiCOR Biosciences, Lincoln, NE, USA).

### 6. Integrity of MPER/Antibody recognition of expressed proteins

The 293T cells were detached from culture and cellular suspensions were incubated with the anti gp41 2F5 or 4E10 monoclonal antibodies (Polymun Scientific, Vienna, Austria) or anti p24 Kc57 monoclonal antibodies (Beckman Coulter, Brea, CA, USA) at a concentration of 4 µg/ml for 20 minutes at room temperature. The level of cell-surface expression was assayed by flow cytometry and by cell-surface and intracellular staining. Unbound antibody was washed in PBS and bound antibodies were revealed using a PE-labeled polyclonal anti human IgG (Jackson ImmunoResearch, West Grove, PA, USA). After a final wash, the cells were analyzed in a LSR II flow cytometer (Becton Dickinson, Franklin Lakes, NJ, USA). The level of expression was determined as the % of positive cells. Non transfected HEK-293T cells were used as controls.

### 7. Determination of residual fusogenic activity

The gp41 subunit of the HIV en glycoprotein has fusogenic activity. This feature has been associated with noxious effects on gp41 expression and bystander cells. In order to determine the residual fusogenic activity of the constructs that may impair immune responses, a fusion assay was performed. HEK-293T cells were transfected with each gp41 coding plasmids in combination with the plasmid pTat (coding for the transactivator HIV protein tat). The cells were cultured with the reporter cell line TZM-bl (AIDS reagent program) 24 hours after transfection. After cell-to-cell fusion, the reporter cell line expressed luciferase under the control of the tat protein. The luciferase activity was measured in a Fluoroskan Accent (Labsystems, Helsinki, Finland) using Britelite Luminescence Reporter Gene Assay System (Perkin Elmer Life Sciences Waltham, MA, USA). The measurements provided a direct estimation of the fusogenic activity of gp41 immunogens. No fusogenic activity was observed, thus ruling out a fusion-related noxious effects of these immunogens.

### 8. VLP immunization protocol

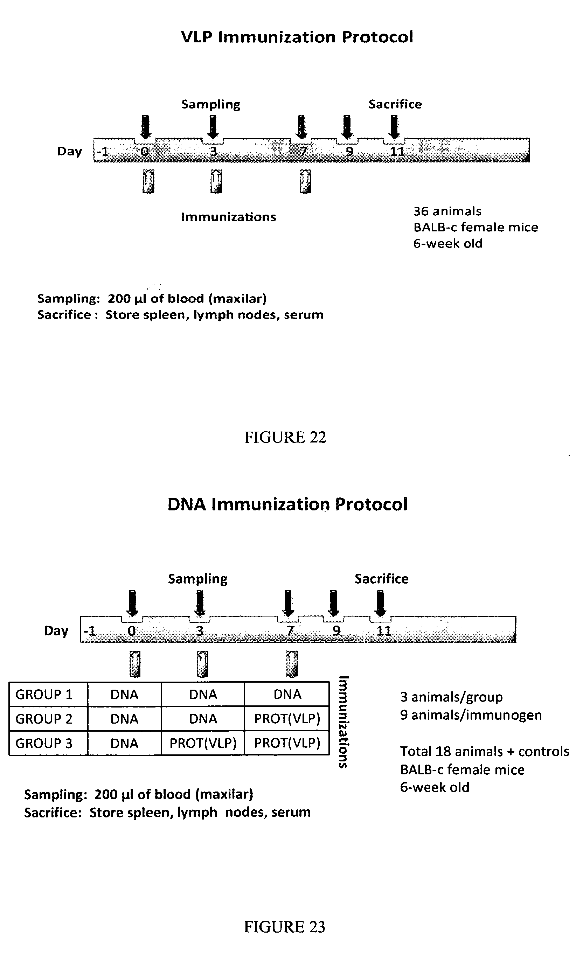

Three groups of BALB-c female mice 6-week old were immunized with the FULL (GP41-MINFULL and GP41-STAPLEFULL) immunogens and the gag VLP control polypeptide at weeks 0, 3 and 7. For each group, a dosage of 5 µg and 20 µg of the immunogen and the polypeptide were applied. The dosages were administered subcutaneously with and without adjuvants (e.g. Al(OH)₃, AlPO₄). 200 µl of maxilar blood samples were collected at weeks 0, 3, 7 and 9. Spleen, lymph nodes and serum samples were collected at week 11. Total antibody responses were characterized by ELISA and ELISPOT assays specific for the gp41 and p24 proteins. *See* Figure 22.

### 9. DNA vaccine preparation and immunization protocol

### A) DNA vaccine preparation

The core FULL (GP41-MINFULL and GP41-STAPLEFULL) immunogen and the gag polypeptide encoding sequences were cloned in a pVAX1 vector (Invitrogen, Carlsbad, CA, USA; Cat. No. V260-20). The signal peptide and intron sequences of the light chain of the murine immunoglobulin G protein were introduced upstream of the FULL immunogen sequences to facilitate expression in murine cells. The expression of the core FULL and gag control vectors was expanded in competent *E coli* with kanamycin selection and purified by using an endotoxin-free purification kit (GenElute™, Sigma-Aldrich, St. Louis, MO, USA).

### B) DNA vaccine immunization protocol

Three groups of BALB-c female mice 6-week old were immunized according to the following procedure:
1) Group 1 was administered 100 µg of the FULL (i.e. GP41-MINFULL, GP41-STAPLEFULL) and the gag VLP control vectors in saline solution intramuscularly at weeks 0, 3 and 7.
2) Group 2 was administered 100 µg of the FULL (i.e. GP41-MINFULL, GP41-STAPLEFULL) and the gag VLP control vectors in saline solution intramuscularly at weeks 0 and 3. In addition, group 2 was administered 5 µg of the FULL (i.e. GP41-MINFULL, GP41-STAPLEFULL) immunogen and the gag VLP control polypeptide with adjuvants (e.g. Al(OH)₃, AlPO₄) subcutaneously at week 7.
3) Group 3 was administered 100 µg of the FULL (i.e. GP41-MINFULL, GP41-STAPLEFULL) and the gag VLP control vectors in saline solution intramuscularly at week 0. In addition, group 3 was administered 5 µg of the FULL (i.e. GP41-MINFULL, GP41-STAPLEFULL) immunogen and the gag VLP control polypeptide with adjuvants (e.g. Al(OH)₃, AlPO₄) subcutaneously at week 3 and 7.

200 µl of maxilar blood samples were collected at weeks 0, 3, 7 and 9. Spleen, lymph nodes, serum and muscle cell samples were collected at week 11. Total antibody responses were characterized by ELISA and ELISPOT assays specific for the gp41 and p24 proteins.

### Example 1

### GP41-MIN Immunogen

A plasmid containing the HV sequence of the isolate HXB2 (pHXB2, Cat. No. 1069, Release Category A) was used as template and amplified with the primers GP41-MIN-f and GP41-r. *See* Figure 4.

The PCR products were then cloned into a pCDNA^{™} vector using the pcDNA™3.1 Directional TOPO® Expression Kit (Invitrogen, Carlsbad, CA, USA; Cat. Nos. K4900-01, K4900-40).

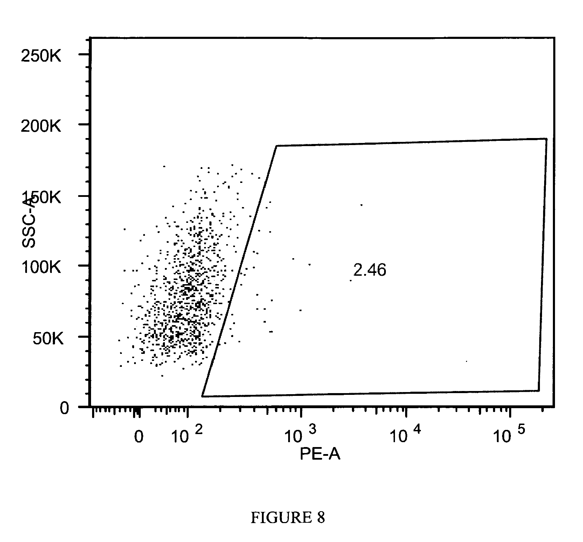

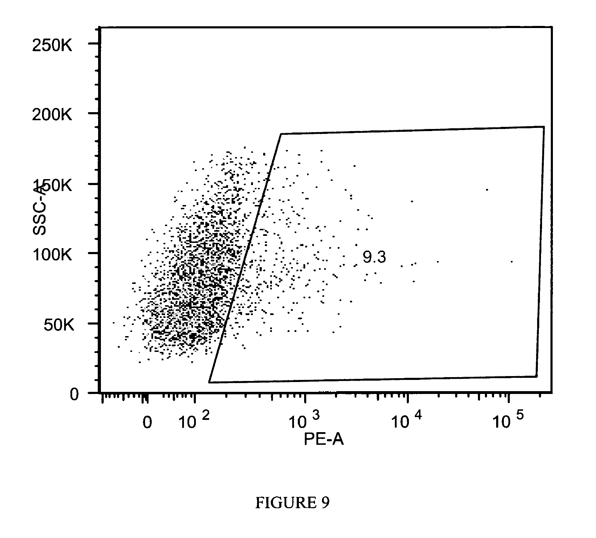

The GP41-MIN immunogen was expressed stably in 293T cells. The immunogen was also recognized by gp41 antibodies. *See* Figures 8 and 9.

### Example 2

### GP41-MINFULL Immunogen

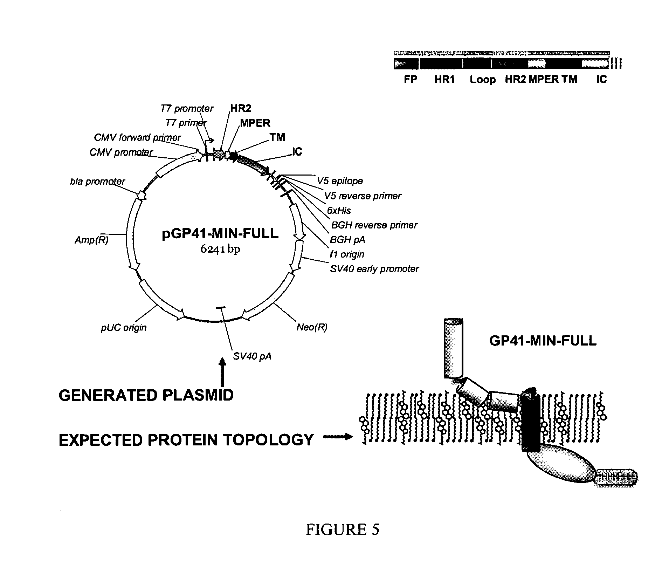

A plasmid containing the HV sequence of the isolate HXB2 was used as template and amplified with the primers GP41-MIN-fand GP41-MAX-r. *See* Figure 5.

The PCR products were then cloned into a pCDNA^{™} vector using the pcDNA™3.1 Directional TOPO® Expression Kit (Invitrogen, Carlsbad, CA, USA; Cat. Nos. K4900-01, K4900-40).

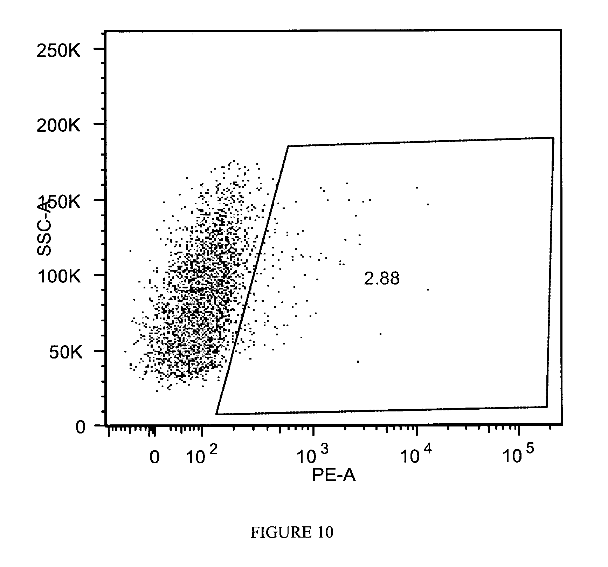

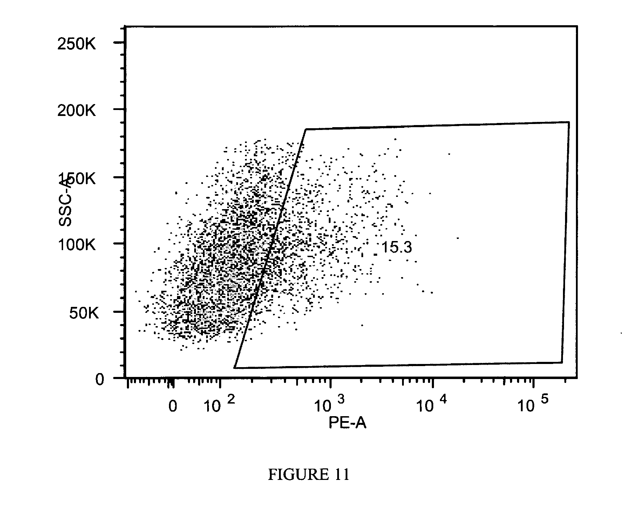

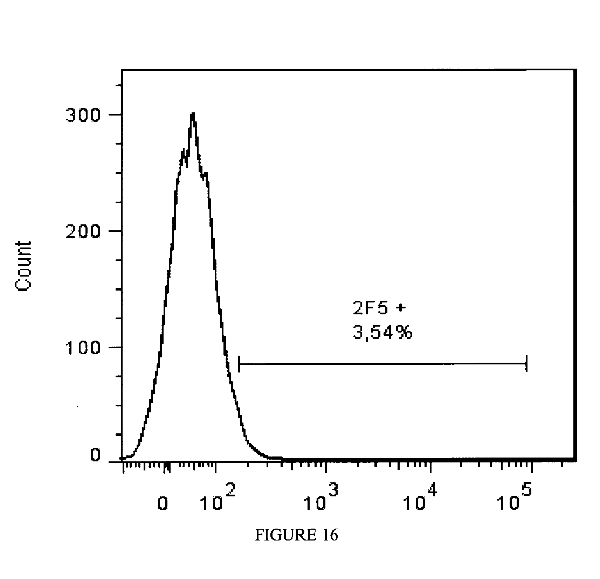

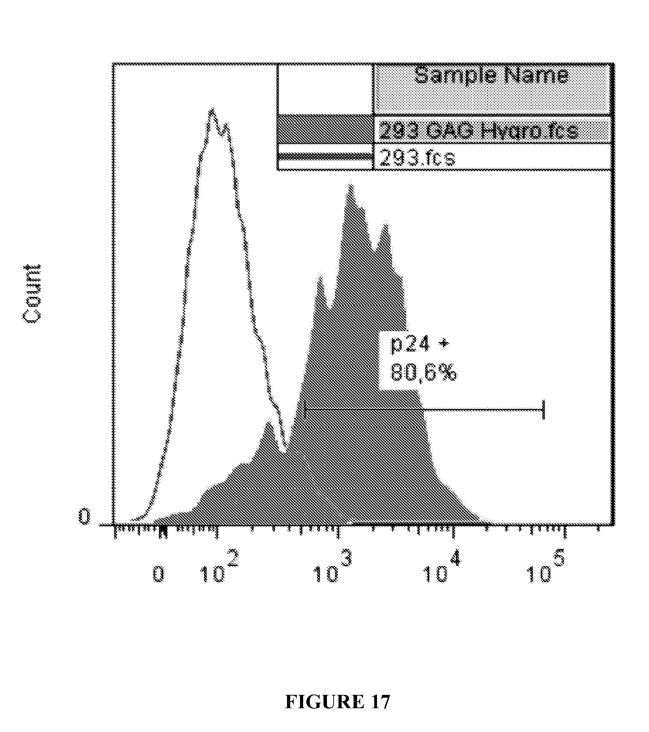

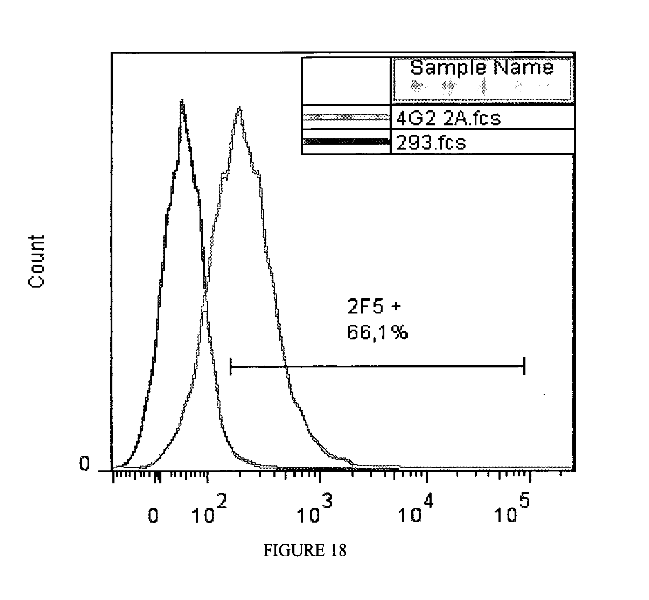

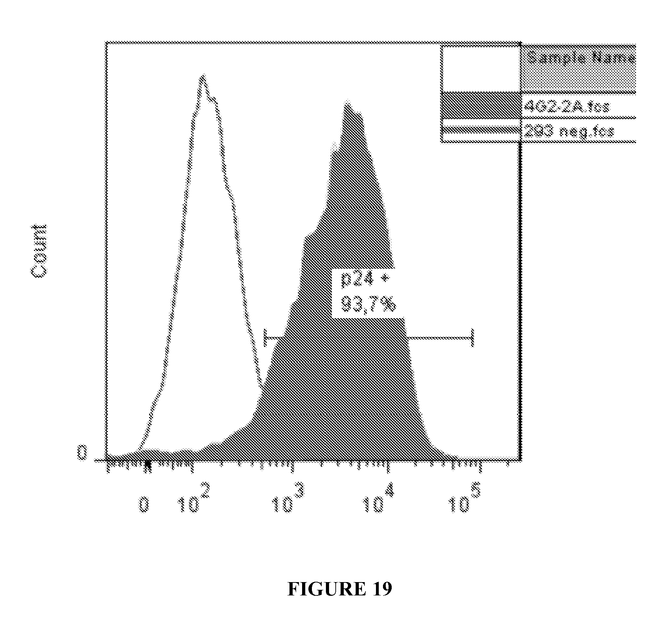

The GP41-MINFULL immunogen and the HIV gag structural protein were expressed stably in 293T cells to produce VLP. The immunogen was also recognized by gp41 antibodies. *See* Figures 10, 11, 16, 17, 18 and 19.

### Example 3

### GP41-STAPLE Immunogen

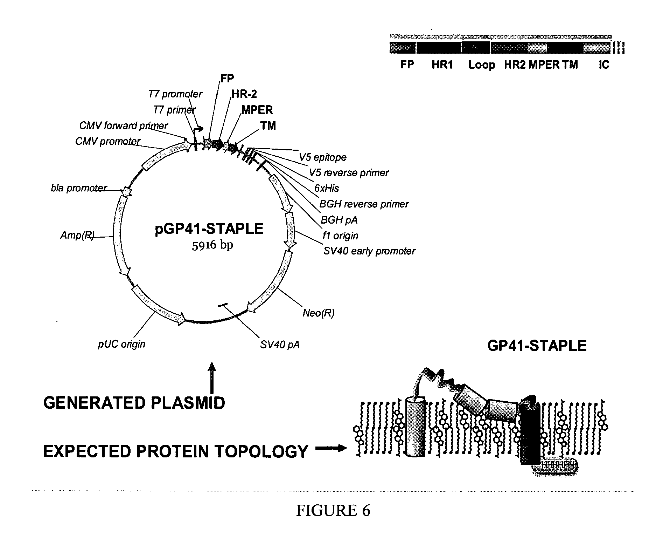

A plasmid containing the HV sequence of the isolate HXB2 was used as template and amplified with the primers GP41-EC-f and FP-r to obtain a fusion peptide coding sequence or with the primers HR-2 f-2 and GP41-r to obtain the other moiety of the construct. A third amplification reaction was performed in a mixture of the above amplified sequences with the primers GP41-EC-f and GP41-MAX-r to obtain the final product. *See* Figure 6.

The PCR products were then cloned into a pCDNA^{™} vector using the pcDNA™3.1 Directional TOPO® Expression Kit (Invitrogen, Carlsbad, CA, USA; Cat. Nos. K4900-01, K4900-40).

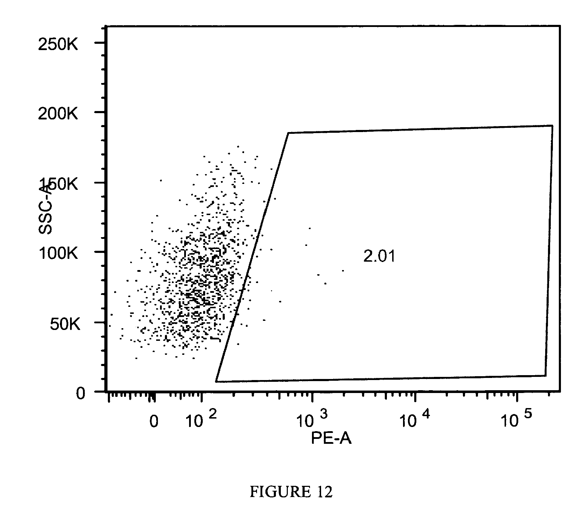

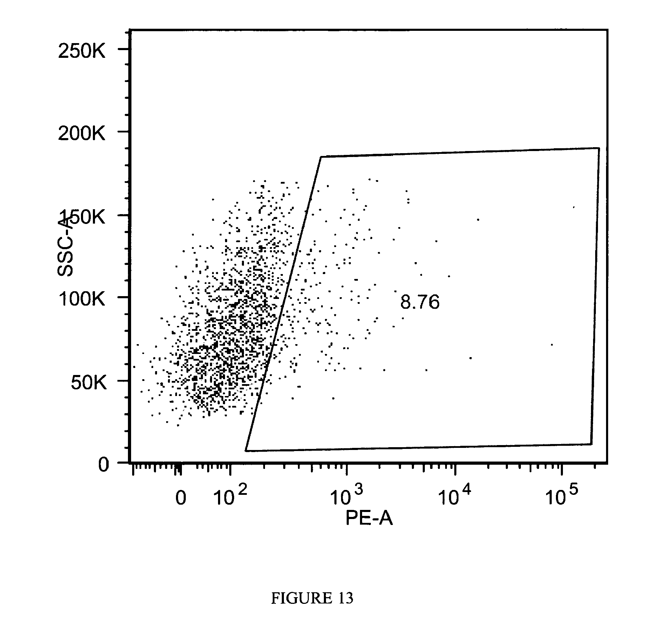

The GP41-STAPLE immunogen was expressed stably in 293T cells. The immunogen was also recognized by gp41 antibodies. *See* Figures 12 and 13.

### Example 4

### GP41-STAPLEFULL Immunogen

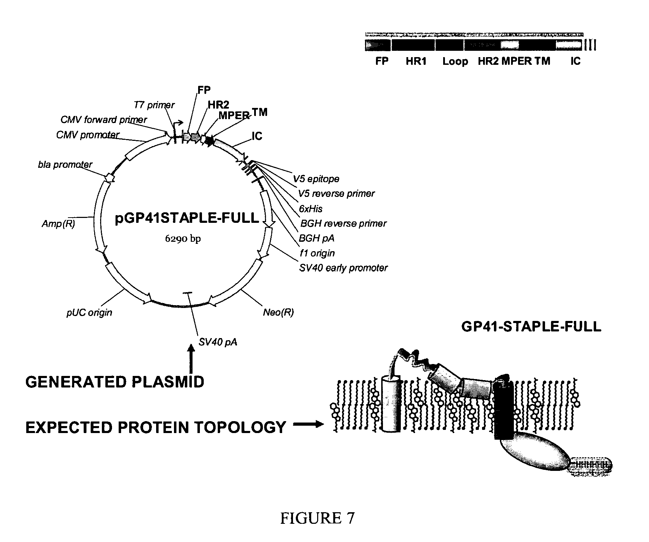

A plasmid containing the HV sequence of the isolate HXB2 was used as template and amplified with the primers GP41-EC-f and FP-r to obtain a fusion peptide coding sequence or with the primers HR-2 f-2 and GP41-MAX-r to obtain the other moiety of the construct. A third amplification reaction was performed in a mixture of the above amplified sequences with the primers GP41-EC-f and GP41-MAX-r to obtain the final product. *See* Figure 7.

The PCR products were then cloned into a pCDNA^{™} vector using the pcDNA™3.1 Directional TOPO® Expression Kit (Invitrogen, Carlsbad, CA, USA; Cat. Nos. K4900-01, K4900-40).

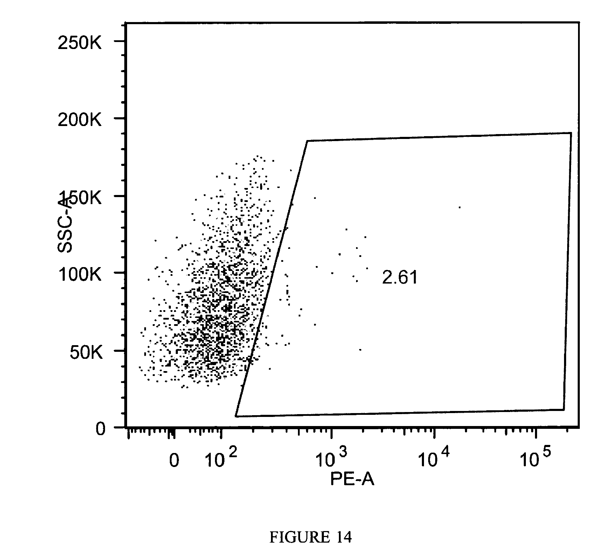

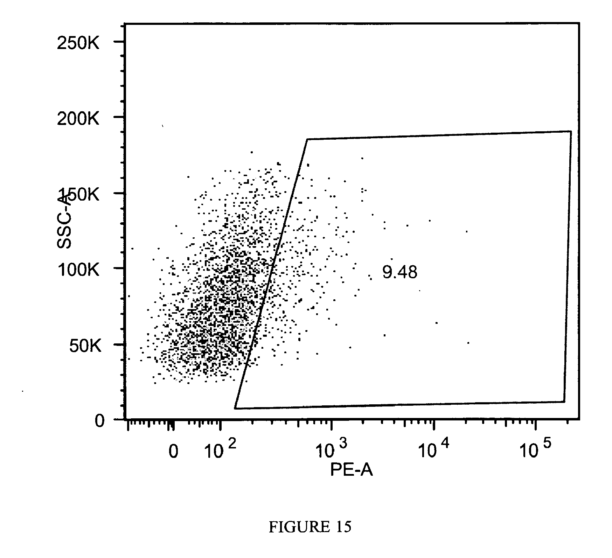

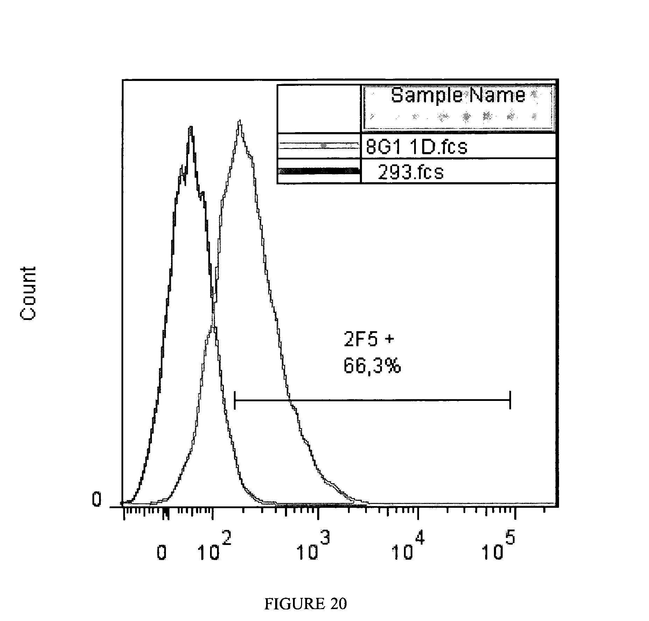

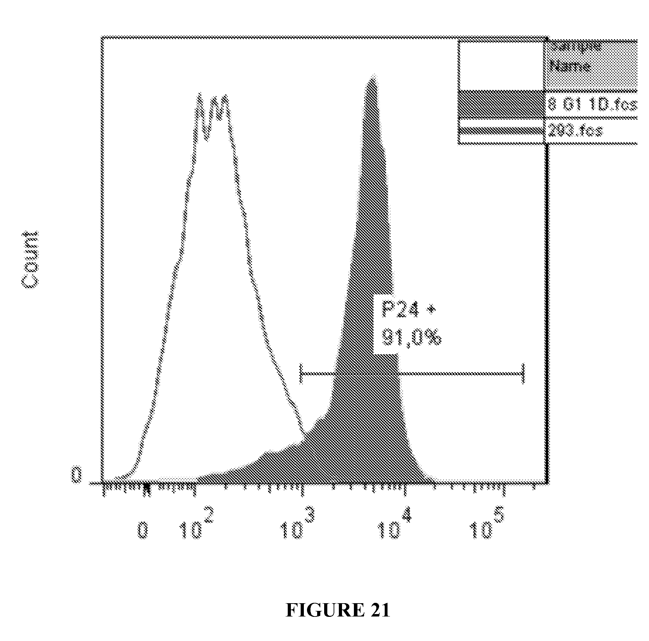

The GP41-STAPLEFULL immunogen and the HIV gag structural protein were expressed stably in 293T cells to produce VLP. The immunogen was also recognized by gp41 antibodies. *See* Figures 14, 15, 16, 17, 20 and 21.

## Claims

1. A HIV gp41 polypeptide variant which lacks at least one of the immunodominant regions in the extracellular domain.

2. A polypeptide according to claim 1 wherein the immunodominant region is selected from the group consisting of the heptad repeat 1, the loop region and the region comprising the heptad repeat 1 and the loop region.

3. A polypeptide according to claims 1 or 2 which comprises from the N- to the C-terminus
(i) the heptad repeat 2 of HIV gp41 or an immunologically equivalent variant thereof,
(ii) the membrane proximal external region of HIV gp41 or an immunologically equivalent variant thereof and
(iii) the transmembrane region of HIV gp41 or an immunologically equivalent variant thereof.

4. A polypeptide according to claim 3 wherein the polypeptide further comprises the intracellular region of HIV gp41 or an immunologically equivalent variant thereof wherein the N-terminus of said intracellular region of HIV gp41 or of the immunologically equivalent variant thereof is connected to the C-terminus of the transmembrane region of HIV gp41 or the immunologically equivalent variant thereof.

5. A polypeptide according to any of claims 3 or 4 wherein the polypeptide further comprises the fusion peptide of HIV gp41 or an immunologically equivalent variant thereof wherein the C-terminus of the said fusion peptide or of the immunologically equivalent variant thereof is connected to the N-terminus of the heptad repeat 2 of HIV gp41 or of the functionally immunologically variant thereof.

6. A polypeptide according to any of claims 1 to 5 wherein the heptad repeat 2 of HIV gp41 comprises amino acids 623 to 664 of the sequence of SEQ ID NO:6, the membrane proximal region of HIV gp41 comprises amino acids 665 to 682 of the sequence of SEQ ID NO:6, the transmembrane region of HIV gp41 comprises amino acids 683 to 705 of the sequence of SEQ ID NO:6, the intracellular region of HIV gp41 comprises amino acids 706 to 856 of the sequence of SEQ ID NO:6 and/or fusion peptide of HIV gp41 comprises amino acids 512-532 of sequence of SEQ ID NO:6.

7. A polypeptide according to claim 6 which comprises a sequence selected from the group consisting of SEQ ID NO:1-18.

8. A polynucleotide encoding a polypeptide as defined in any of claims 1 to 7.

9. A polynucleotide according to claim 8 wherein the polynucleotide comprises a sequence selected from the group consisting of SEQ ID NO: 19 to 30.

10. A vector comprising a polynucleotide as defined in claims 8 or 9.

11. A host cell comprising a polypeptide as defined in any of claims 1 to 7, a polynucleotide as defined in claims 8 or 9 or a vector as defined in claim 10.

12. A vaccine composition comprising an immunogenically active amount of a polypeptide as defined in any of claims 1 to 7, a polynucleotide as defined in claims 8 or 9, a vector as defined in claim 10 or a host cell as defined in claim 11.

13. A polypeptide as defined in any of claims 1 to 7, a polynucleotide as defined in claims 8 or 9, a vector as defined in claim 10 or a host cell as defined in claim 11 for use in medicine.

14. A polypeptide as defined in any of claims 1 to 7, a polynucleotide as defined in claims 8 or 9, a vector as defined in claim 10 or a host cell as defined in claim 11 for use in the treatment of HIV infection.

15. A method for the detection of anti-gp41 antibodies in a sample which comprises contacting said sample with a polypeptide according to any of claims 1 to 7 under conditions adequate for the formation of a complex between the antibodies in the sample and the polypeptide and detecting the formation of said complex.
